# EUROPEAN PATENT SPECIFICATION

(11) **EP 2 503 334 B1**
(45) Date of publication and mention of the grant of the patent: **31.12.2014**
(21) Application number: 12166847.9
(22) Date of filing: 06.04.2010
(51) Int. Cl.: G01N 33/50

(54) **Identification of regulatory t cells via the global gene regulator SatB1**
Identifizierung von regulatorischen T-Zellen über den globalen Genregulator SatB1
Identification de lymphocytes T régulateurs via le régulateur de gène global SatB1

(30) Priority: 02.04.2009 US 165970 P
(43) Date of publication of application: 26.09.2012
(62) Divisional of application: 10715520.2
(73) Proprietor: Becton Dickinson and Company, Franklin Lakes, NJ 07417 (US); Rheinische Friedrich-Wilhelms-Universität Bonn, 53113 Bonn (DE)
(72) Inventor: Schultze, Joachim Ludwig, 53639 Königswinter (DE); Beyer, Marc Daniel, 50935 Köln (DE); Warner, Noel, Los Gatos, CA California 95050 (US); Balderas, Robert, San Diego, CA California 92121 (US)
(74) Representative: Von Kreisler Selting Werner - Partnerschaft von Patentanwälten und Rechtsanwälten mbB

(56) References cited:
- US-A1- 2008 280 298
- PFOERTNER SUSANNE ET AL: "Signatures of human regulatory T cells: an encounter with old friends and new players", GENOME BIOLOGY, BIOMED CENTRAL LTD., LONDON, GB LNKD- DOI:10.1186/GB-2006-7-7-R54, vol. 7, no. 7, 12 June 2006 (2006-06-12), page R54, XP021021257, ISSN: 1465-6906
- LI ET AL: "SATB1 regulates SPARC expression in K562 cell line through binding to a specific sequence in the third intron", BIOCHEMICAL AND BIOPHYSICAL RESEARCH COMMUNICATIONS, ACADEMIC PRESS INC. ORLANDO, FL, US LNKD- DOI:10.1016/J.BBRC.2007.01.201, vol. 356, no. 1, 17 March 2007 (2007-03-17), pages 6-12, XP005926975, ISSN: 0006-291X
- LECHNER O ET AL: "Fingerprints of anergic T cells.", CURRENT BIOLOGY : CB 17 APR 2001 LNKD- PUBMED:11369203, vol. 11, no. 8, 17 April 2001 (2001-04-17) , pages 587-595, XP002588557, ISSN: 0960-9822
- LOMBARDI G ET AL: "Anergic T cells as suppressor cells in vitro.", SCIENCE (NEW YORK, N.Y.) 10 JUN 1994 LNKD- PUBMED:8202711, vol. 264, no. 5165, 10 June 1994 (1994-06-10), pages 1587-1589, XP002588558, ISSN: 0036-8075
- LING LU ET AL: "Characterization of protective human CD4CD25 FOXP3 regulatory T cells generated with IL-2, TGF-[beta] and retinoic acid", PLOS ONE, PUBLIC LIBRARY OF SCIENCE, US, vol. 5, no. 12, 17 December 2010 (2010-12-17), pages 1-12, XP002652171, ISSN: 1932-6203, DOI: 10.1371/JOURNAL.PONE.0015150
- TAKAHISA MIYAO ET AL: "Plasticity of Foxp3T Cells Reflects Promiscuous Foxp3 Expression in Conventional T Cells but Not Reprogramming of Regulatory T Cells", IMMUNITY, CELL PRESS, US, vol. 36, no. 2, 23 December 2011 (2011-12-23), pages 262-275, XP028461586, ISSN: 1074-7613, DOI: 10.1016/J.IMMUNI.2011.12.012 [retrieved on 2012-01-24]

## Description

The present invention provides a method for the identification of unstable regulatory T cells based on the elevated levels of the global gene regulator SATB1 in regulatory T cells. The invention also relates to a method utilizing ligands that specifically bind to SATB1 for identifying unstable regutatory T cells.

### Background of the Invention

Regulatory T cells (T_{reg}) are involved in self tolerance, immune homeostasis, prevention of autoimmunity, and suppression of immunity to pathogens or tumours (Sakaguchi, S. et al., Cell 133:775-787 (2008); Balkaid, Y., Nat. Rev. Immunol 7:875-888 (2007); Beyer, M., Schultze, J., Blood 1008:804-811 (2006)). The forkhead transcription factor FOXP3 is essential for T_{reg} development and function as mutations in FOXP3 cause severe autoimmunity in mice and humans (Hori, S. et al., Science 299:1057-1061 (2003); Fontenot, J.D et al., Nat. Immunol. 4:330-336 (2003); Khattri, R. et al., Nat. Immunol. 4:337-342 (2003); Brunkow, M.E. et al., Nat. Genet. 27:68-73 (2001); Bennett, C.L. et al., Nat. Genet. 27:20-21 (2001); Wildin, R.S. et al., Nat. Genet. 27:18-20 (2001)). FOXP3 prevents effector T cell (T_{effector}) lineage commitment (Zhou, L. et al., Nature 453:236-240 (2008)), yet, the underlying molecular mechanisms are still elusive.

T_{reg} are characterized by their suppressive function and inability to produce cytokines. Expression of FOXP3 is required for the establishment and maintenance of T_{reg} lineage, identity and suppressor function (Hori, S. et al., Science 299:1057-1061 (2003); Fontenot, J.D et al., Nat. Immunol. 4:330-336 (2003); Khattri, R. et al., Nat. Immunol. 4:337-342 (2003); Lin, W. et al., Nat. Immunol. 8:359-368 (2007); Wan, Y.Y., Flavell, R.A., Nature 445:766-770 (2007); Lahl, K. et al., J. Immunol. in press (2009); Williams, L. M., Rudensky, A.Y., Nat. Immunol. 8:277-284 (2007)). Loss of FOXP3 in T_{reg} has been associated with a TH2 (Lin, W. et al., Nat. Immunol. 8:359-368 (2007); Wan, Y.Y., Flavell, R.A., Nature 445:766-770 (2007); Lahl, K. et al., J. Immunol. in press (2009)) respectively TH1 (Lin, W. et al., Nat. Immunol. 8:359-368 (2007)) or TH17 (Gavin, M.A. et al., Nature 445:771-775 (2007)) phenotype of T_{reg} suggesting that FOXP3 actively suppresses differentiation of T_{reg} into T_{effector}. One mechanisms of repression of T_{effector} differentiation by FOXP3 might be the direct modulation of transcription factors (Ziegler, S.F., Annu. Rev. Immunol. 24:209-226 (2006)), such as interferon regulatory factor-4 (IRF4), which is necessary for T_{reg}-mediated suppression of TH2 effector cell differentiation (Zheng, Y. et al., Nature (2009)). Epigenetic control by DNA methylation or histone modification has been suggested as an alternative mechanism sustaining T_{reg} phenotype and function (Wei, G. et al., Immunity 30:155-167 (2009)). These novel findings indicate that there is a significant degree of plasticity between T_{effector} and T_{reg} lineages and that active, regulatory mechanisms must enable committed T_{reg} to prevent T_{effector} differentiation. The present invention provides novel marker genes for the specific identification and characterization of human suppressive and/or regulatory T cells including natural, adaptive, and expanded CD4⁺CD25⁺FOXP3⁺ T cells in healthy individuals as well as tumor patients or patients with autoimmune diseases.

Pfoertner et al. (GenomeBiology 2006, 7:R54) describes genetic signature of regulatory T cells.

### Short description of the Invention

It was now found that the chromatin remodelling enzyme SATB1 (SEQ ID NO:2) which is required for normal thymic T-cell development (Alvarez, J.D. et al., Genes Dev. 14:521-535 (2000)), peripheral T-cell homeostasis (Nie, H. et al., J. Immunol. 174:4745-4752 (2005)), TH1/TH2 polarization (Cai, S. et al. Nat. Genet. 38:1278-1288 (2006); Lund, R. et al., Eur. J. Immunol. 35:3307-3319 (2005)), and reprogramming of gene expression (Han, H.J. et al., Nature 452:187-193 (2008)) is an important target gene of FOXP3. SATB1 expression is significantly reduced in natural and induced murine and human FOXP3⁺ T_{reg}. FOXP3 reduces SATB1 expression directly as a transcriptional repressor at the SATB1 locus and indirectly via the induction of microRNAs miR-155, miR-21, miR-7, miR-34, and miR-18a, specifically binding to the 3'UTR of the SATB1 mRNA. Reduced SATB1 expression in FOXP3⁺ cells achieved either by overexpression or induction of FOXP3 is linked to significant reduction in TH1 and TH2 cytokines. Loss of FOXP3 function either by knock down or genetic mutation leads to significant upregulation of SATB1 and subsequent cytokine release. Since the SATB1 locus is similarly demethylated in T_{reg} and T_{effector}, T_{reg} lineage commitment requires FOXP3-mediated active and continuing inhibition of SATB1 thereby prohibiting T_{effector} differentiation. This places SATB1-mediated T cell-specific modulation of global chromatin remodelling central during the decision process between effector and regulatory T-cell function. More importantly, overexpression of SATB1 in human natural T_{reg} cells leads to loss of suppressive function and gain of T_{effector} function in T_{reg} cells. These data strongly suggest that inhibition of SATB1-mediated, T-cell specific modulation of global chromatin remodelling is centrally important for the control of functional plasticity in T_{reg} cells. The invention thus provides
1. A method of detecting unstable regulatory T cells in a population of regulatory T cells that have the potential for converting to effector T cell functionality, which method comprises detecting cells with elevated levels of SATB1 protein expression in the population of T cells.
2. The method of item 1, wherein the cells with elevated levels of SATB1 protein expression in the population of T cells are detected by a method comprising
   (a) contacting the cell population with one or more ligands that specifically bind to SATB1, and
   (b) identifying the regulatory T cells in the cell population due to a significant reduction of binding with the SATB1-binding ligands as compared to binding of said ligands with the other cells in the cell population.
3. The method of item 1 or 2, which is suitable for quality control of regulatory T cell populations.
4. The method of item 2, wherein the ligands are antibodies or fragments thereof, preferably the ligands are monoclonal antibodies or fragments thereof.
5. The method of item 2 or 4, wherein the ligands/antibodies carry functional moieties including, but not limited to labels, dyes and toxins.
6. The method of item 2, 4 or 5, wherein the cell population is selected from cell culture, whole blood and fractions of whole blood and/or the cell population comprises mammalian cells including human cells.
7. The method of item 6, further comprising contacting the human cell population with one or more ligands that specifically bind to CD4, CD25 and/or CD127 on the T cells.
8. The method of item 6 or 7, further comprising assaying for FOXP3 expression.
9. Use of the ligand, the antibody or antibody fragment of item 2, 4 or 5 for identifying unstable regulatory T cells in a cell population.

### Short description of the Figures

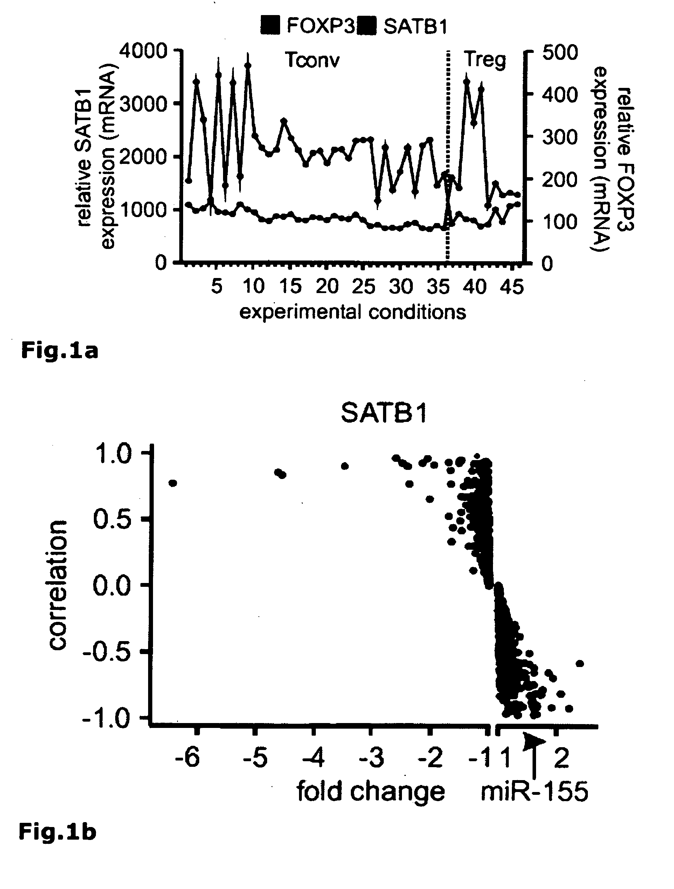

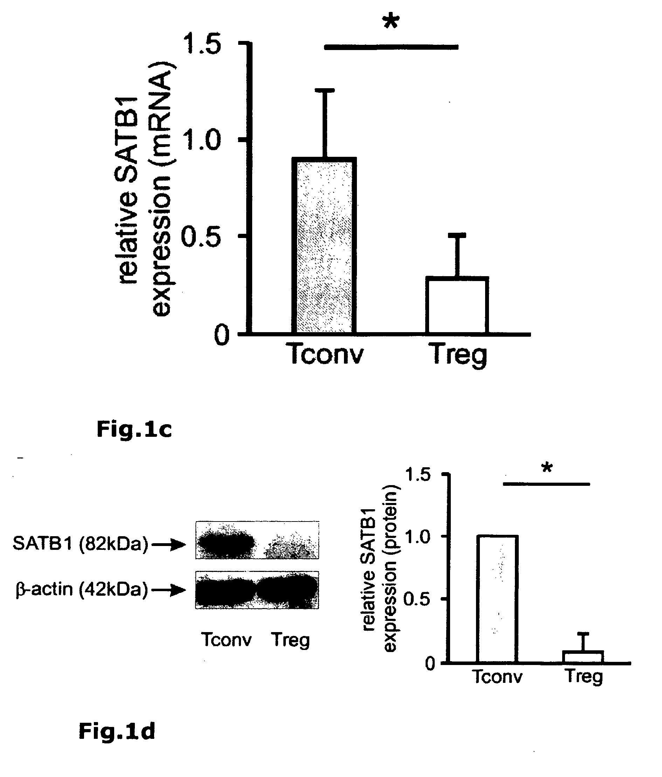

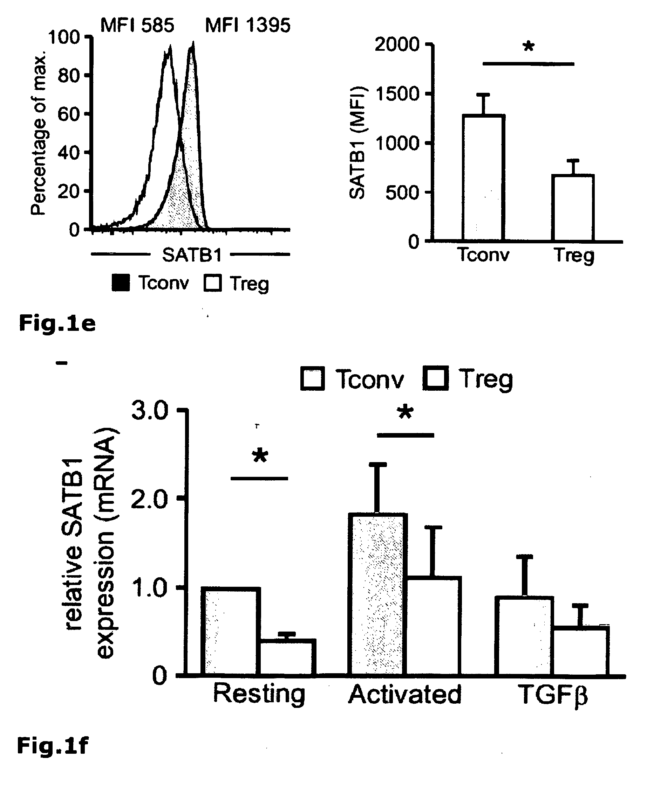

Fig. 1: SATB1 is downregulated in human natural regulatory T cells. CD4⁺CD25^{high} CD127^{low} natural regulatory T cells (T_{reg}) were purified either by FACS or MACS sorting (>95% purity). Conventional CD4⁺CD25⁻ T cells (T_{conv}) were used for comparison. At least 4 donors were studied and mean +/- SD is depicted; * p< 0.05. a: SATB1 (red) and FOXP3 (blue) mRNA expression as assessed by microarray analysis in a total of 48 experimental conditions (3-4 replicates each, see Table 1). The individual conditions are grouped here according to experimental condition (act=activation, rest=resting, exp=expanded)) and lineage (CD4⁺CD25⁺ T_{reg} (T_{reg}) vs. CD4⁺CD25⁻ T cells (CD25⁻) vs. total CD4⁺ T cells (T_{conv})). b: Correlation of miRNA expression with SATB1 mRNA expression is plotted against miRNA fold change (T_{reg} vs. T_{conv}) for all 735 miRNA assessed. Highlighted in red is miR-155. c: relative SATB1 mRNA expression in T_{reg} and T_{conv} assessed by qRT-PCR (mean +/- SD, 6 individual experiments were performed). d: Westernblot analysis of SATB1 protein expression in a representative donor (left) and relative expression of SATB1 (n=3, mean +/- SD, right). e: Intracellular staining for SATB1 in a representative experiment (left) and mean SATB1 expression +/- SD in T_{reg} and T_{conv} (n=11, right). f: Influence of TCR activation, costimulation and TGFβ stimulation on SATB1 mRNA expression in T_{conv} and T_{reg} assessed by qPCR after cultivation for 72 h (mean +/- SD, 5 individual experiments were performed).

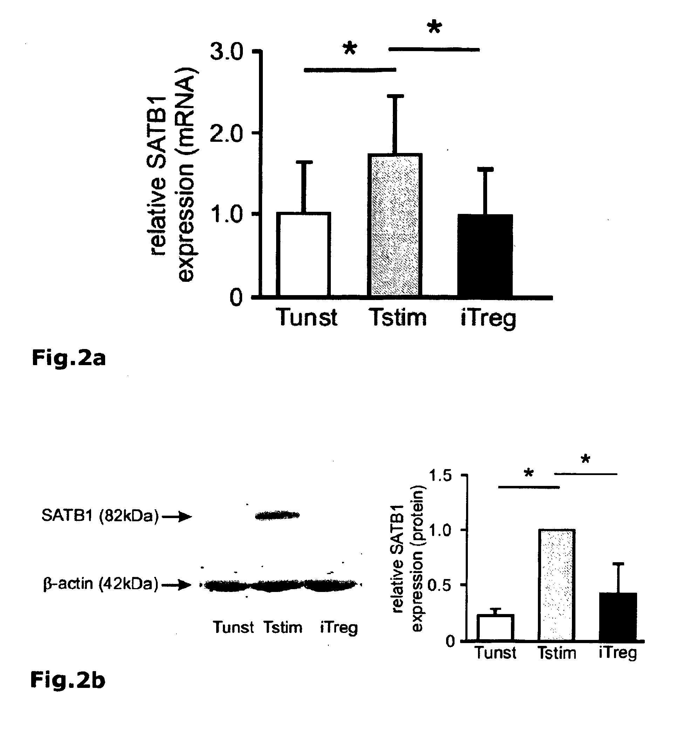

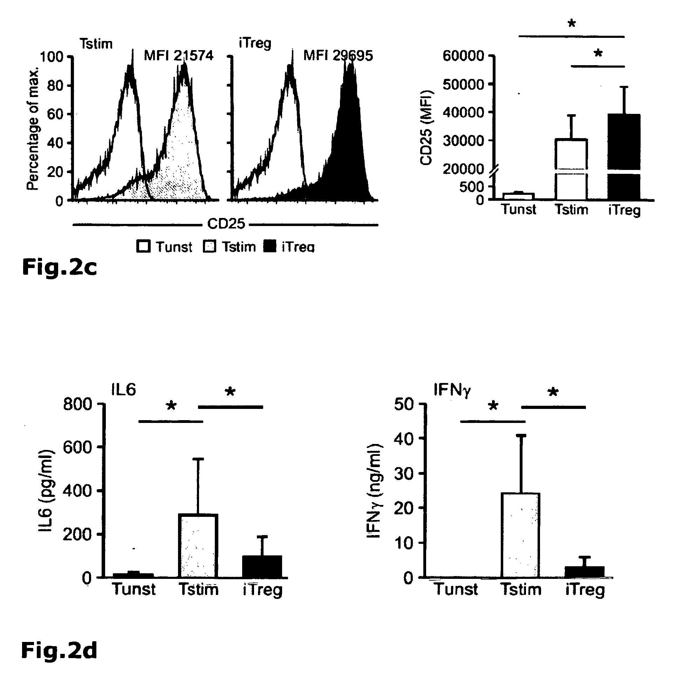

Fig. 2: SATB1 is downregulated during induction of human regulatory T cells. Naïve human CD45RA⁺CCR7⁺CD4⁺ T cells were either left unstimulated (Tᵤₙₛₜ), stimulated with CD3 and CD28 beads (Tₛₜᵢₘ) or stimulated in the presence of TGFß to become induced regulatory T cells (iT_{reg}). At least 3 donors were studied and mean +/- SD is depicted; * p< 0.05. a: SATB1 mRNA expression (mean +/- SD) as assessed by qRT-PCR after 5 d (n= 6). b: Westernblot analysis of SATB1 protein expression in one representative experiment (left) and relative expression of SATB1 (n= 3, mean +/-SD, right). c: Flow cytometric analysis of CD25 protein expression in Tₛₜᵢₘ, and iT_{reg} for one representative donor (left) and mean CD25 expression +/- SD (right) after 5 days (n=4). d: CBA assessment of IL6 and IFN-γ cytokine secretion (n=7, mean +/- SD).

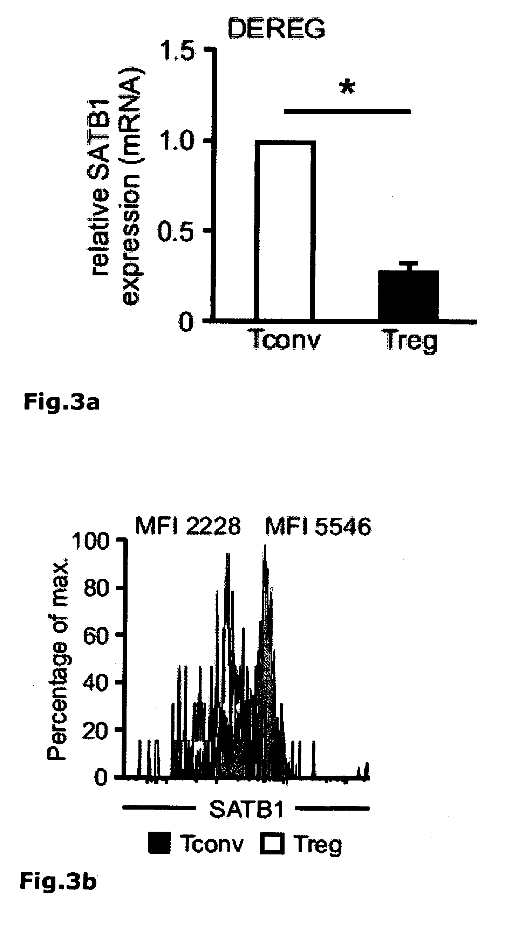

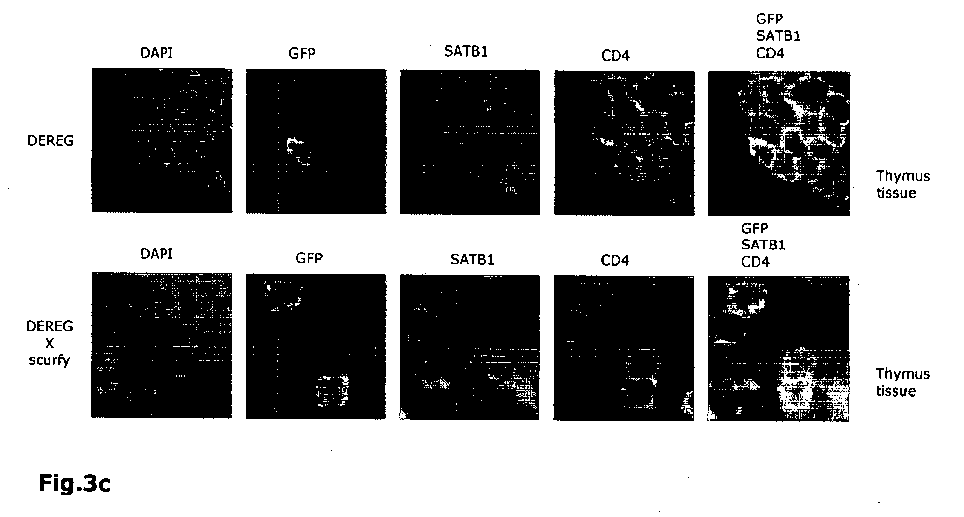

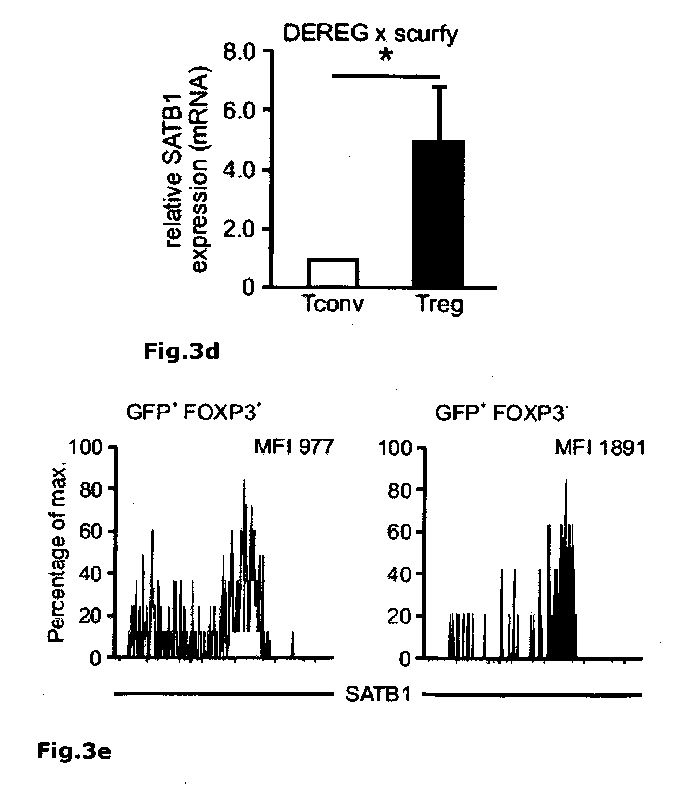

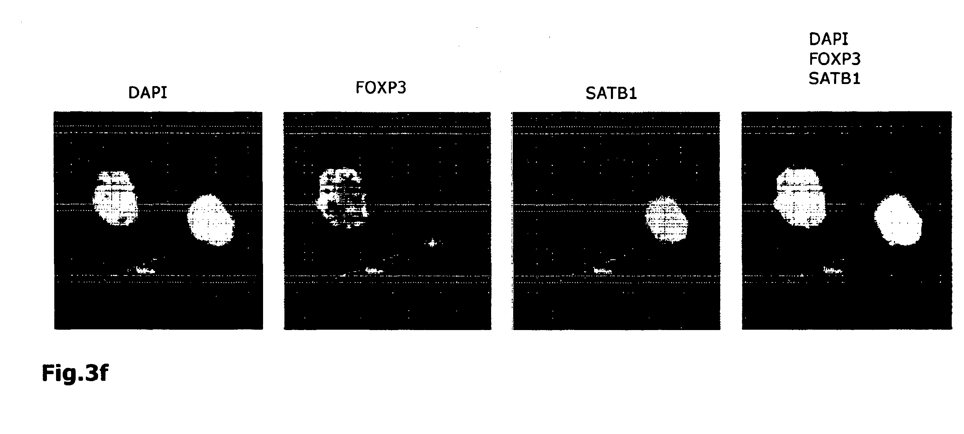

Fig. 3: SATB1 is dysregulated *in vivo* in FOXP3-deficient T_{reg} cells from DEREG mice. a: Analysis of SATB1 mRNA expression (mean +/- SD; * p< 0.05) in T_{conv} and T_{reg} derived from male DEREG mice. A representative of two independent experiments is shown. b: Intracellular staining for SATB1 in a representative experiment in T_{reg} and T_{conv} from DEREG mice. c: Immunofluorescent staining of thymocytes for SATB1 protein expression (red) in GFP⁺ T_{reg} (green) counterstained with DAPI (blue) and CD4 (magenta) from male DEREG and FOXP3-deficient DEREG mice (DEREG x scurfy) as assessed by quadruple staining. d: SATB1 mRNA expression (mean +/- SD; * p< 0.05) in T_{conv} and T_{reg} derived from male FOXP3-deficient DEREG x scurfy mice assessed by qRT-PCR. A representative of two independent experiments is shown. e: Flow cytometric analysis of intracellular SATB1 protein expression in FOXP3-sufficient (FOXP3⁺, left) and -deficient (FOXP3⁻, right) GFP⁺ T_{reg} from female DEREG mice heterozygous for the scurfy mutation. f: Immunofluorescent staining for SATB1 (red) and FOXP3 (green) protein expression in GFP⁺ T_{reg} sorted from thymus tissue of female DEREG mice heterozygous for the scurfy mutation counterstained with DAPI (blue).

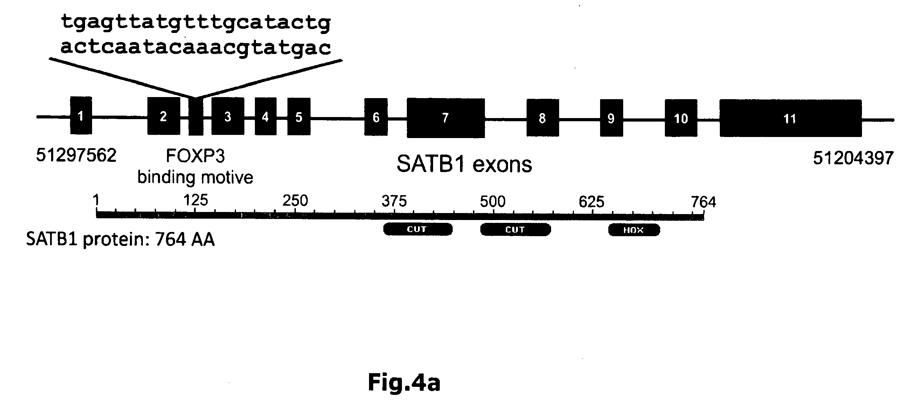

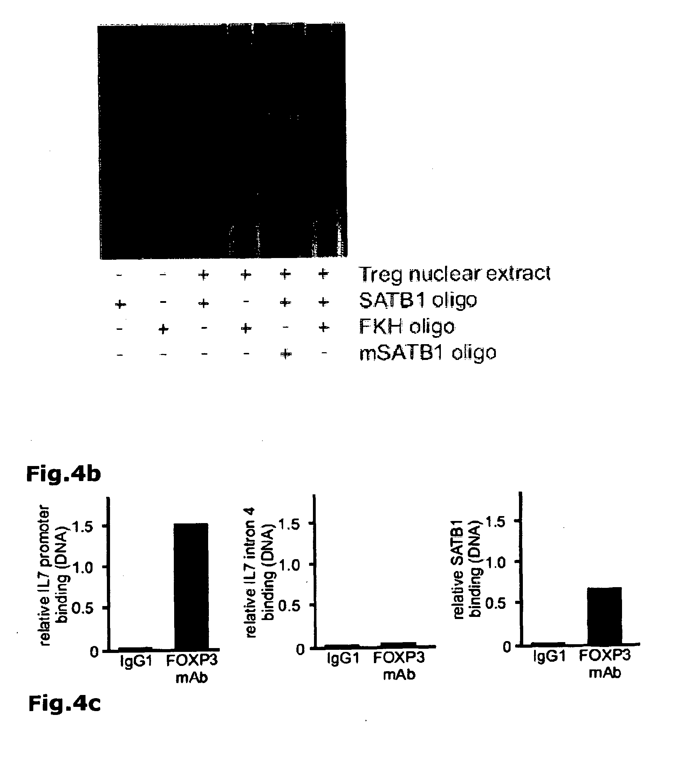

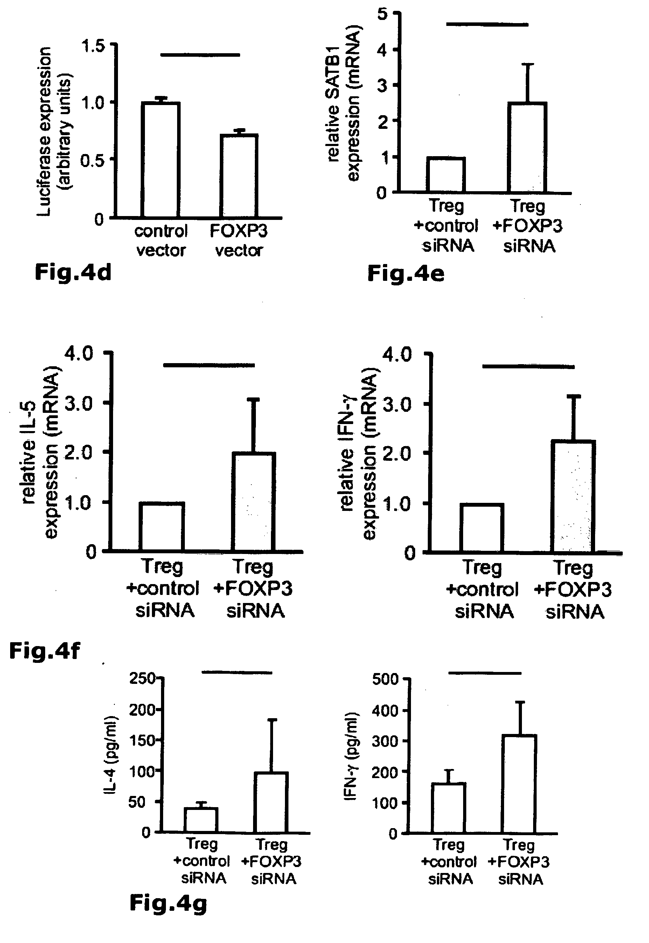

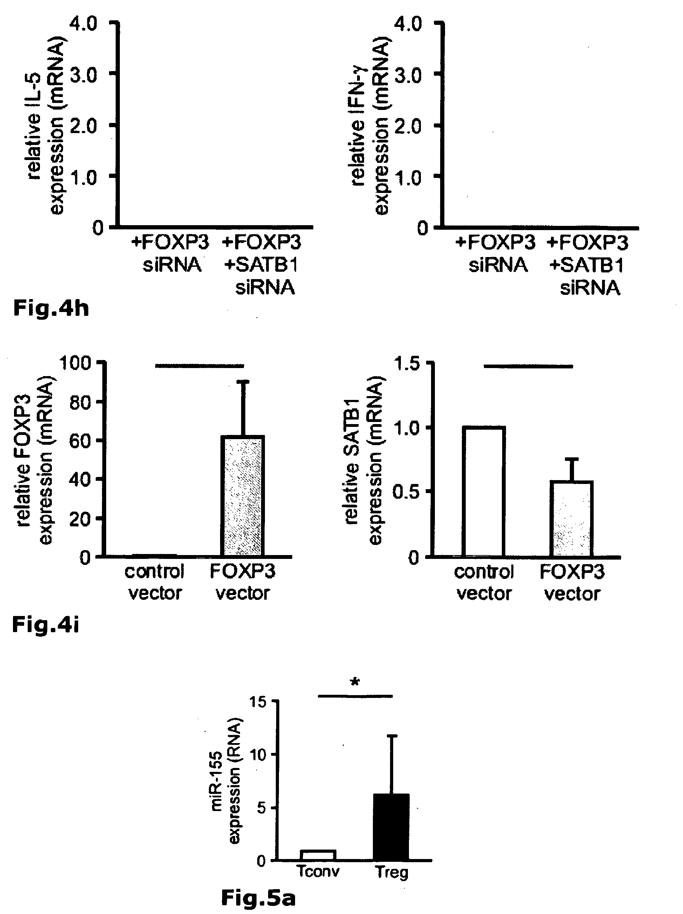

Fig. 4: Direct suppression of SATB1 mRNA transcription by FOXP3. a: Representation of the human genomic SATB1 genomic region and the conserved FOXP3 binding site. b: Electromobility shift assay (EMSA) assessing FOXP3 binding to the genomic SATB1 region (intron 2). Nuclear extracts from expanded human natural T_{reg}; SATB1 oligo: specific nucleotide for the FOXP3-binding site in the genomic SATB1 region; FKH Oligo: nucleotide containing the general forkhead motive; mSATB1 Oligo: mutated nucleotide for the FOXP3-binding site in the genomics SATB1 region. c: FOXP3 binding to the genomic SATB1 region in intron 2 assessed by ChIP-qPCR. PCR was performed using a primer set corresponding to the SATB1 intron 2 region and FOXP3 antibody or control IgG precipitated chromatin isolated from expanded human natural T_{reg}. IL7R promoter locus was used as a positive, the IL7R intron 4 region as negative control. Shown here is one representative experiment of 2. d: Luciferase activity was assessed by luminometric analysis after transfection of a reporter construct containing the potential FOXP3 binding site in the genomic SATB1 region in intron 2 or with a mutated motive into HEK293 cells. FOXP3 binding was assessed in comparison between cells transfected with a control or FOXP3-expressing vector (mean +/- SD; * p<0.05) in comparison to the mutated motive. A representative of three independent experiments is shown. e-g: MACS-purified human natural T_{reg} were either transfected with a scrambled control siRNA or FOXP3-specific siRNA and assessed 48 h post knockdown. e: SATB1 mRNA expression (mean +/- SD; n=6, * p<0.05) in FOXP3-sufficient (control siRNA) and -deficient (FOXP3 siRNA) primary human natural T_{reg} assessed by qRT-PCR. f: Analysis of IL-5 and IFN-γ mRNA expression (n=4, mean +/- SD) in FOXP3-sufficient (control siRNA) and -deficient (FOXP3 siRNA) primary human natural T_{reg} assessed by qRT-PCR. g: CBA assessment of IL-4 and IFN-γ cytokine secretion (triplicates, mean +/- SD) of FOXP3-sufficient (control siRNA) and -deficient (FOXP3 siRNA) primary human natural T_{reg} (n=4). h: Assessment of IL-5 and IFN-γ mRNA expression (mean +/- SD) in primary human natural T_{reg} transfected with SATB1-specific siRNA after silencing of FOXP3 48 hours post knockdown. i: Analysis of FOXP3 (left) and SATB1 (right) expression in human conventional T_{reg}-depleted CD4⁺ T cells lentivirally transfected with FOPX3 by qRT-PCR (mean +/- SD, n=5, * p<0.05).

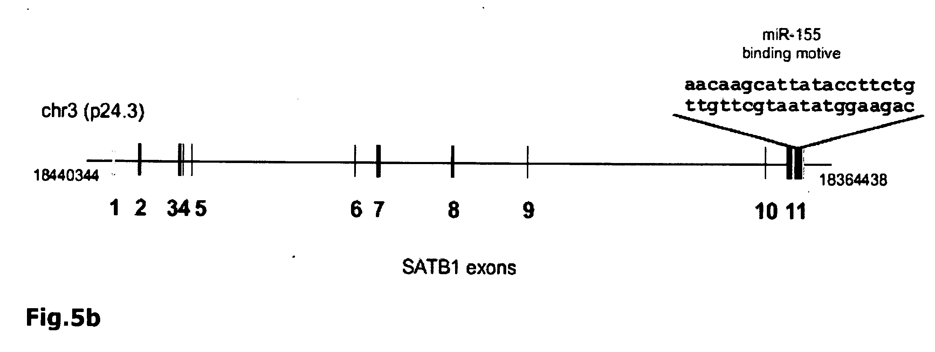

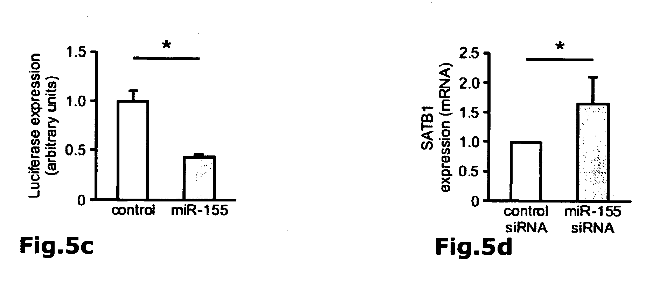

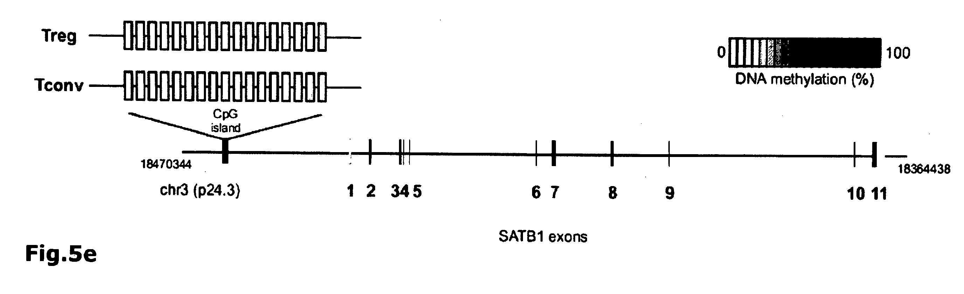

Fig. 5: Regulation of SATB1 by miRNA. a: Mean miR-155 expression in human natural T_{reg} in comparison to T_{conv} (mean +/- SD; n=7, * p<0.05) as assessed by qPCR. b: Representation of the human genomic SATB1 genomic region and the conserved miR-155 binding site in the 3' UTR (SEQ ID NO:43). c: Luciferase activity was assessed by luminometric analysis after transfection of a reporter construct containing the SATB1 3' UTR into HEK293 cells. Regulation of SATB1 expression by miR-155 was assessed by transfection of miR-155 in comparison with a scrambled control miRNA (mean +/-SD; * p<0.05). Mutation of the miR-155 motif was used to demonstrate specificity. A representative of three independent experiments is shown. d: MACS-purified human natural T_{reg} were either transfected with a miR-122 siRNA (control) or miR-155-specific siRNA and regulation of SATB1 mRNA expression assessed 48 h post knockdown by qRT-PCR (mean +/- SD, n=5, * p<0.05). f: DNA methylation of the predicted CpG-island in the genomic region of SATB1 in T_{reg} and T_{conv}.

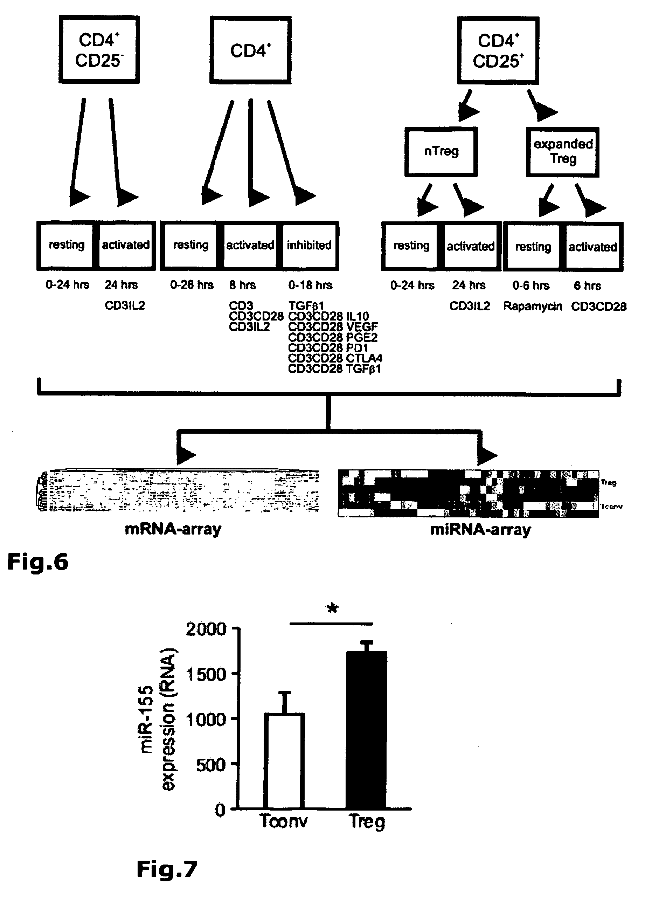

Fig. 6: Layout of microarray experiments performed to identify SATB1 expression and microRNA regulation in T_{reg}. Human CD4⁺ T cells, CD4⁺ CD25⁻ T_{conv}, CD4⁺ CD25⁺ T_{reg}, and expanded T_{reg} were assessed either directly after isolation (resting), after up to 24 h of cell culture without further stimulation (resting), or after activation by various stimuli (activated). Included are also inhibitory conditions of CD4⁺ T cells stimulated in the presence of inhibitory signals including IL10, prostaglandin-E2 (PGE2), PD1, CTLA-4 or TGFβ1. If not otherwise indicated cells were stimulated for 8 h prior harvesting for microarray analysis (see also Table 1).
Fig. 7: Assessment of miR-155 by array analysis. Mean miR-155 expression in human nT_{reg} in comparison to T_{conv} as assessed by miRNA microarray analysis. At least 3 donors were studied and mean +/- SD is depicted; * p<0.05.

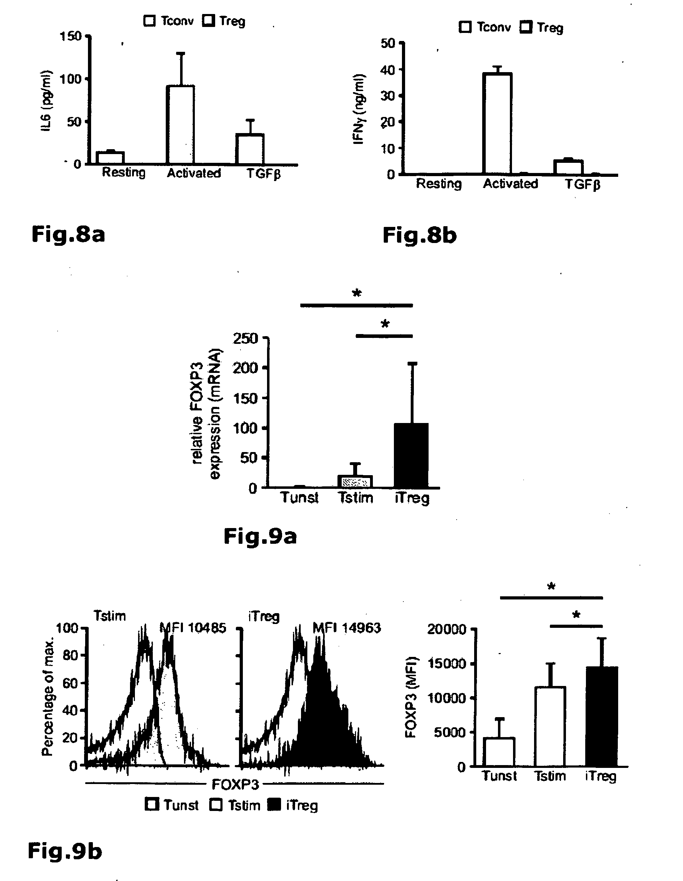

Fig. 8: Influence of activation and TGFß stimulation on TH1/TH2 cytokine secretion in T_{conv} and T_{req}. CD4⁺ CD25^{high} CD127^{low} nT_{reg} were purified by MACS sorting (>96% purity). CD4⁺ CD25⁻ T_{conv} were used for comparison. Influence of activation (CD3 + CD28 beads) and TGFβ stimulation on IL6 and IFN-γ release in T_{conv} (grey bars) and T_{reg} (white bars) was assessed by cytometric bead arrays. Cells were cultured for 72 h, 4 donors were studied and mean +/- SD is depicted; * p< 0.05.

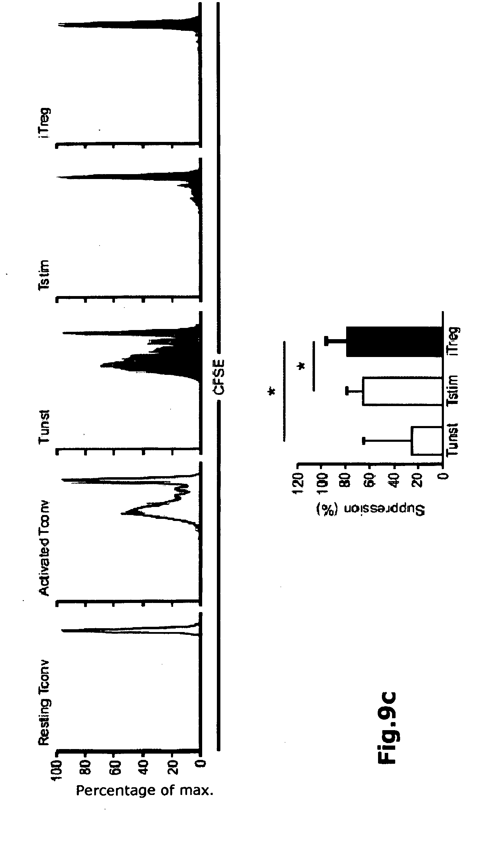

Fig 9: FOXP3 expression and suppressive function of induced regulatory T cells. Naïve human CD45RA⁺CCR7⁺CD4⁺ T cells were either left unstimulated (Tᵤₙₛₜ), stimulated with CD3 and CD28 beads (Tₛₜᵢₘ) or stimulated in the presence of TGFβ to become induced regulatory T cells (iT_{reg}). At least 3 donors were studied and mean +/- SD is depicted; * p< 0.05. a: FOXP3 mRNA (mean +/- SD) expression as assessed by qRT-PCR after 5 d (n= 6). b: Intracellular staining of FOXP3 in a representative experiment (left) and mean FOXP3 expression +/- SD (right) after 5 d (n= 4). c: Flow cytometric analysis of regulatory function of Tᵤₙₛₜ, Tₛₜᵢₘ, and iT_{reg} as assessed by inhibition of proliferation of allogeneic CD4⁺ T cells at a 1:1 ratio; shown here CFSE staining of one representative experiment (left) and mean suppressive activity +/- SD (n=9) of Tᵤₙₛₜ, Tₛₜᵢₘ, and iT_{reg} (right). Resting conventional CD4⁺ T cells served as negative control, allogeneic T cells stimulated with CD3 and CD28 beads as positive control.

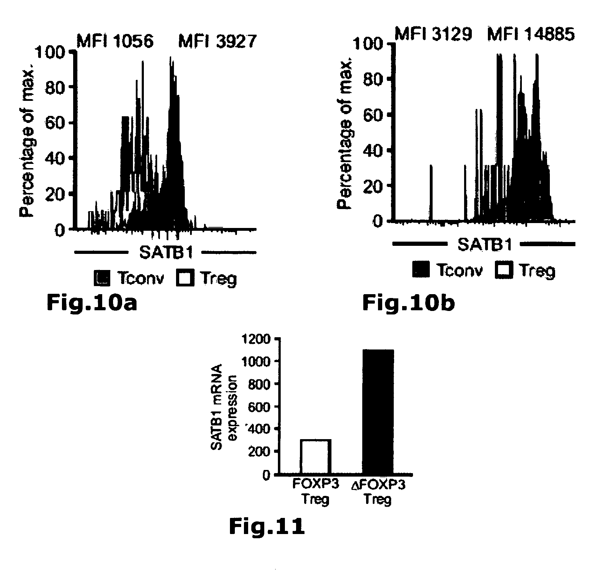

Fig. 10: Flow cytometric analysis of SATB1 expression in DEREG mice. T_{reg} as well as T_{conv} from DEREG mice were stained for CD4, FOXP3, and SATB1 and gated on CD4, GFP, and FOXP3 expression. SATB1 expression in T_{reg} and T_{conv} cells from lymphnodes (a) and thymus (b) was assessed by flow cytometry. MFI values are presented in the upper left resp. right corner for T_{reg} resp. T_{conv}.
Fig. 11: Microarray analysis of SATB1 expression in T_{reg}, from ΔFOXP3 mice. Microarray data of Williams, L. M. & Rudensky, A. Y., Nat Immunol 8, 277-284 (2007) were reanalyzed for SATB1 expression in T_{reg} cells and FOXP3 knockout T_{reg}.

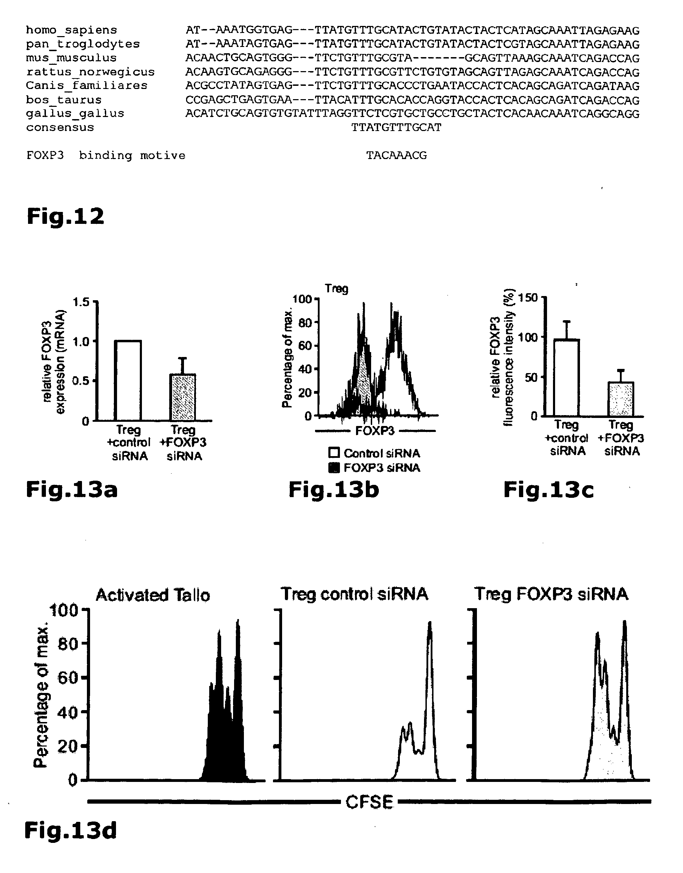

Fig. 12: Conservation of the FOXP3-binding site in the SATB1 locus over several mammals (SEQ ID NOs:35-42). Sequence alignment was performed using ClustalW.

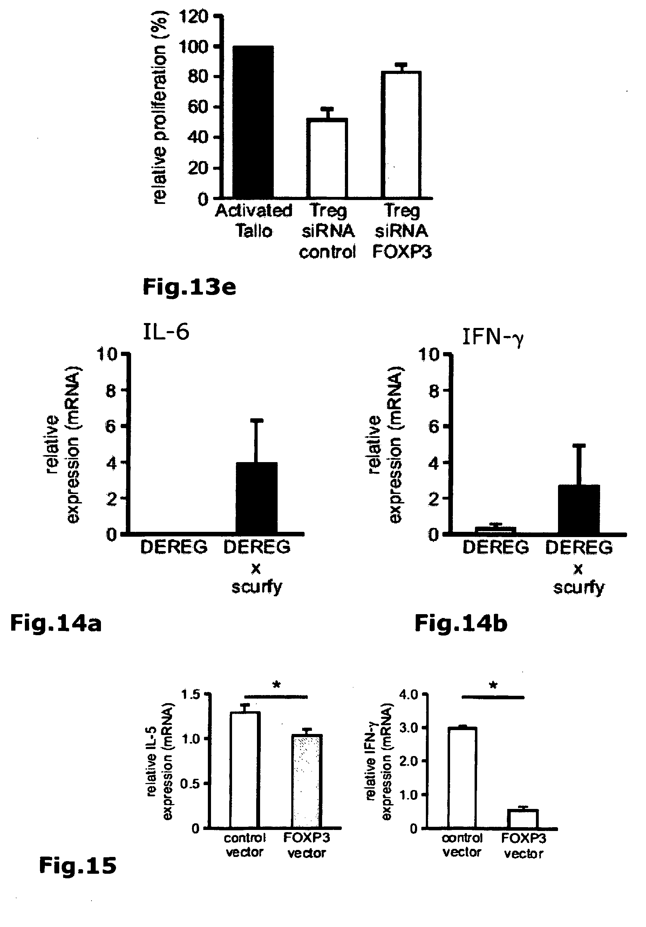

Fig.13: Knockdown of FOXP3 in primary human T_{reg.}_ Human T_{reg} were either transfected with control siRNA or FOXP3-specific siRNA and assessed 48 hours post knockdown. a: relative FOXP3 mRNA expression (mean +/- SD, n=6, * p<0.05). b: Representative flow cytometric analysis of intracellular FOXP3 expression 48 hours post FOXP3 knockdown in T_{reg}. c: Mean FOXP3 protein expression (mean +/- SD, n=6, * p<0.05). d: Suppressive function of control or FOXP3 siRNA treated T_{reg} assessed in a standard suppressive assay using CD4⁺ allogeneic T cells as readout. One representative experiment is shown. e: Mean inhibitory capacity (mean +/- SD, n=3, * p<0.05).
Fig. 14: TH1/TH2 differentiation of T_{reg} from DEREG x scurfy mice. To assess whether T_{reg} expressing SATB1 differentiate in T-helper cells expressing TH1/TH2 cytokines, we isolated GFP⁺ T_{reg} and analyzed IL-6 (a) and IFN-γ (b) mRNA production by T_{reg} derived from DEREG or DEREG x scurfy mice. A representative of two independent experiments is shown.
Fig. 15: T_{conv} transfected with FOXP3 show reduced cytokine production. Analysis of IL-5 (left) and IFNγ (right) expression in human conventional T_{reg}-depleted CD4⁺ T cells lentivirally transfected with FOPX3 by qRT-PCR (mean +/- SD, n=5, * p<0.05).

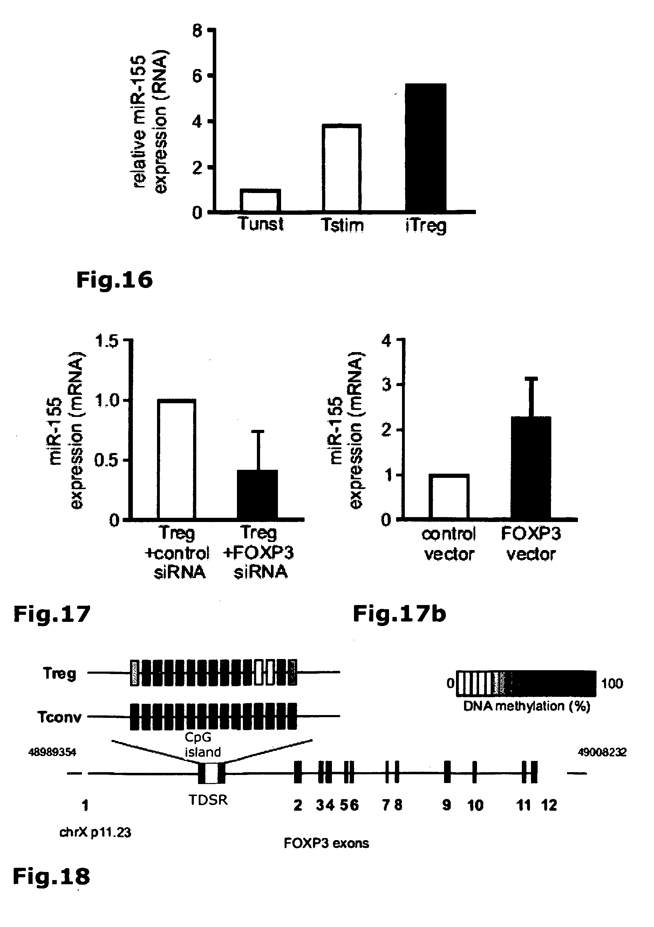

Fig. 16: MiR-155 is highly expressed in human iT_{reg}. Analysis of miR-155 expression in Tᵤₙₛₜ, Tₛₜᵢₘ, and iT_{reg} cells by miRNA-specific PCR.
Fig. 17: MiR-155 is a downstream target of FOXP3 in human T cells. a: Regulation of miR-155 after knockdown of FOXP3 in human nT_{reg} was analyzed by miRNA-specific PCR in comparison to a control siRNA. b: After lentiviral transduction of CD4+ T_{conv} with either FOXP3 or a control vector miR-155 expression was assessed by miRNA-specific PCR.
Fig. 18: DNA methylation of the CpG island of the FOXP3 locus in T_{reg} and T_{conv}.

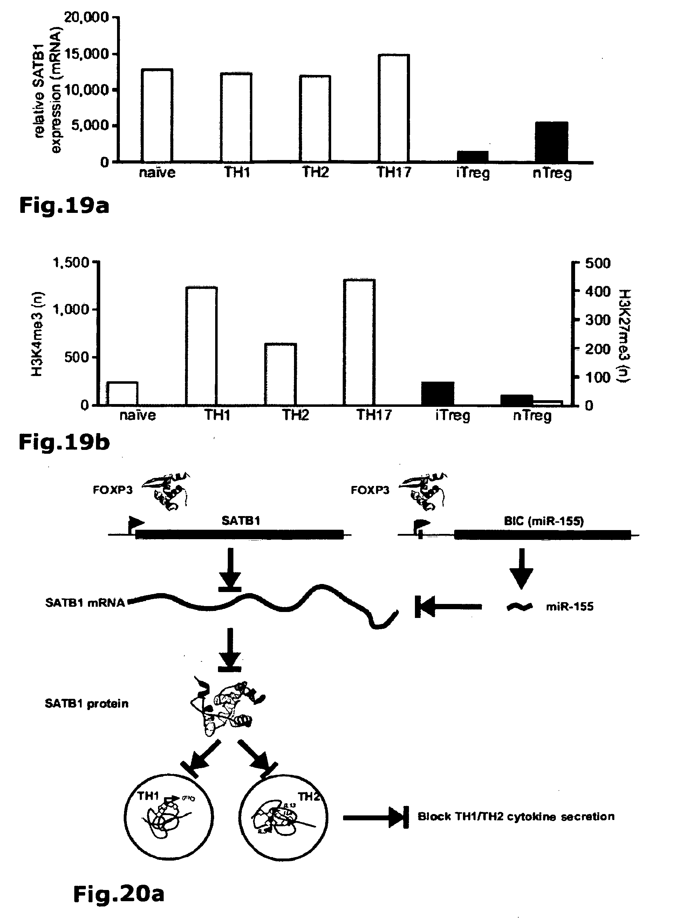

Fig. 19: Histone methylation at the SATB1 gene locus. Data published by Wei, G. et al., Immunity 30:155-167 (2009) were reanalyzed for SATB1 expression and histone methylation maps in murine naive T cells, T_{effector} (ThH1, TH2, resp. TH17), iT_{reg} and nT_{reg}. a: expression of SATB1 as assessed by microarray analysis. b: ChIP-sequencing data were re-analyzed for the SATB1 locus. Trimethylation of H3K4 is associated with gene activation, whereas di- and trimethylation of H3K27 are associated with gene repression. In none of the T cell subsets trimethylation of H3K27 was detected while T_{effector} showed high levels of H3K4 methylation and T_{reg} lower methylation.

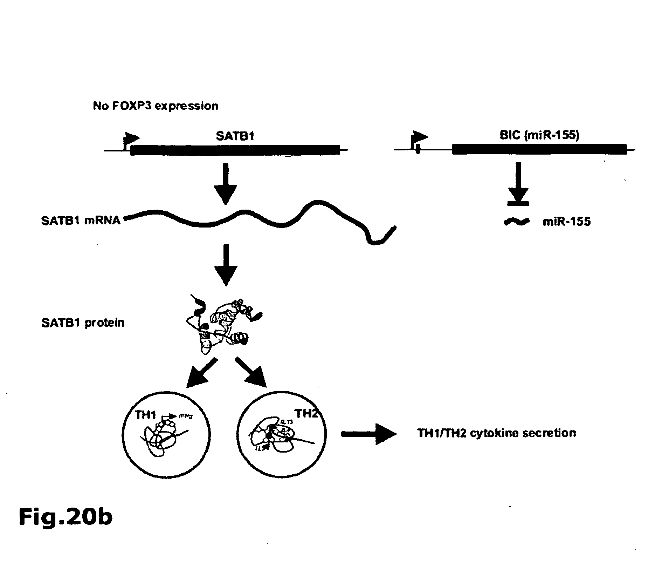

Fig. 20: Model for the mode of action of FOXP3 and miR155 on the SATB1 protein expression and downstream TH1 and TH2 cytokin secretion.

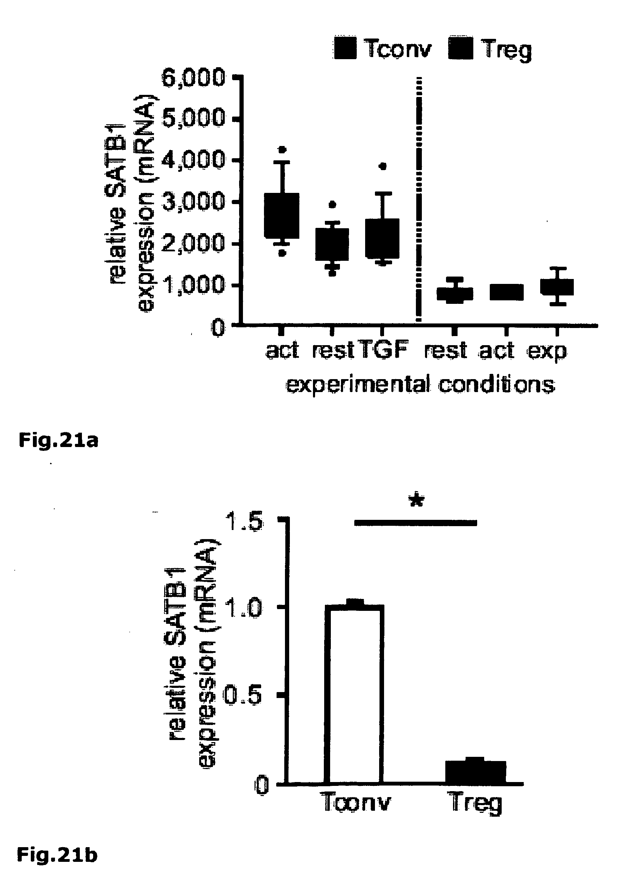

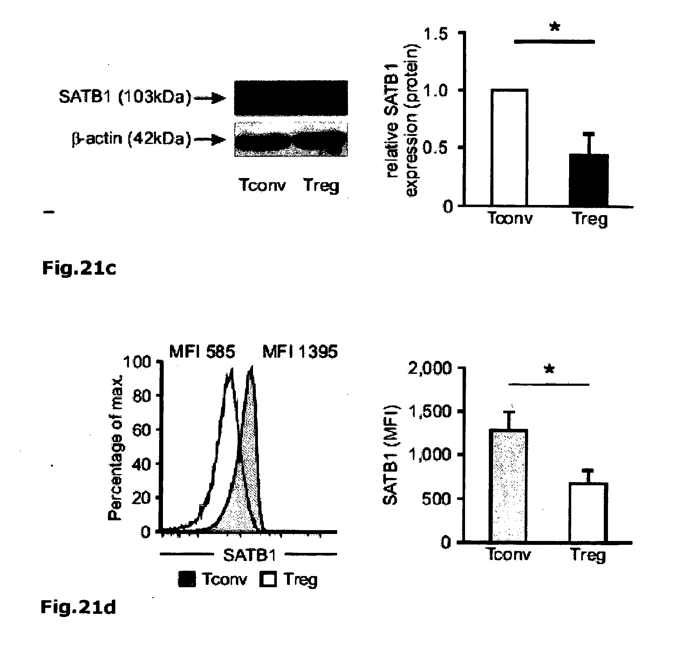

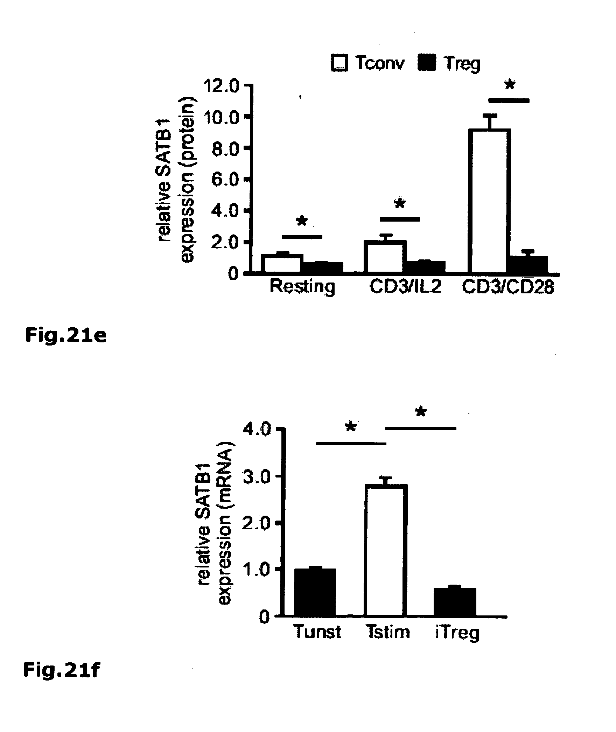

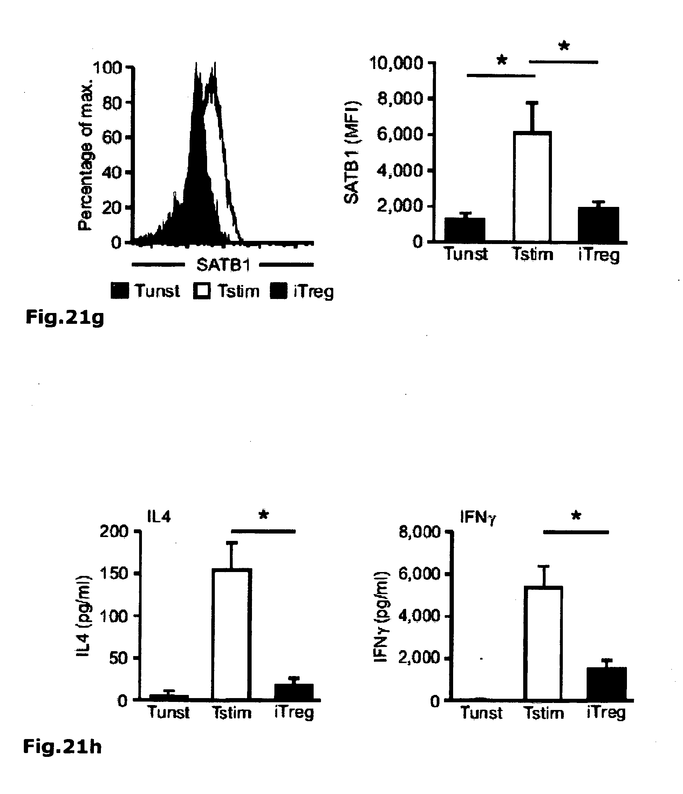

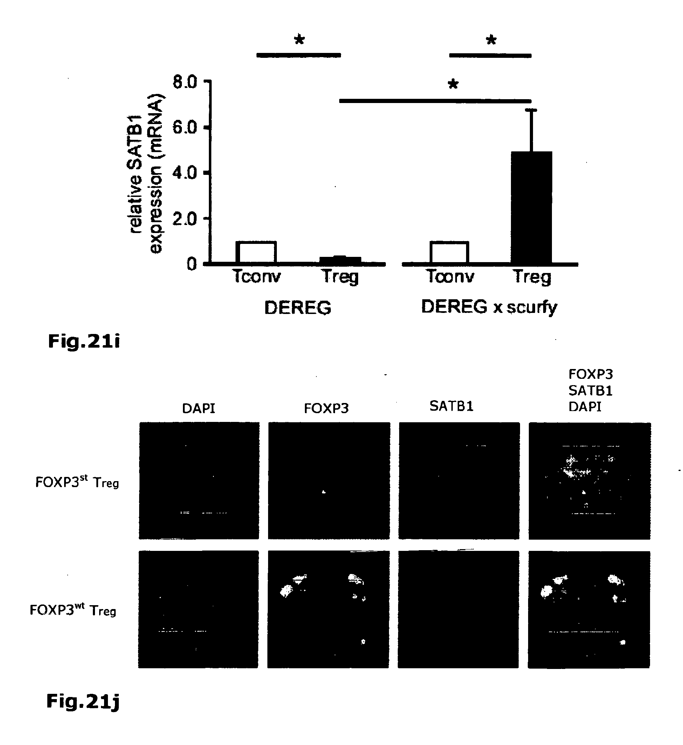

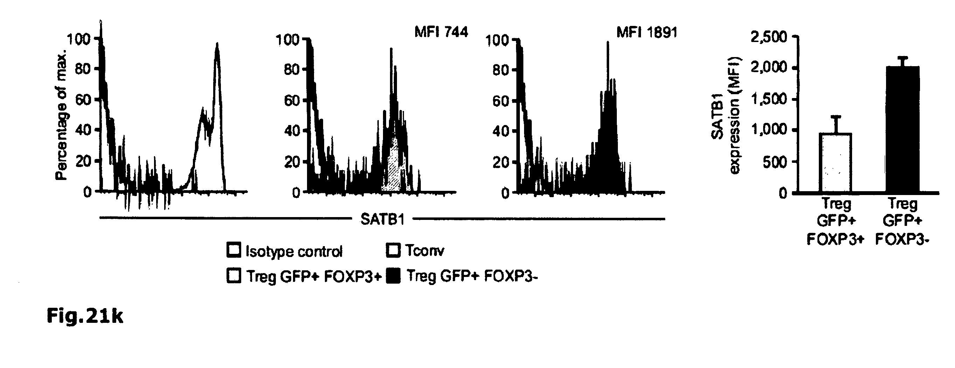

Fig. 21: FOXP3-dependent repression of SATB1 expression in regulatory T cells. (a) microarray analysis of SATB1 mRNA expression in conventional T cells (T_{conv}, blue) and regulatory T cells (T_{reg}, red) under different conditions; act = activated via TCR for 8 h, rest = no activation, TGF = stimulation with TGFβ for 8 h, exp = expanded with TCR and costimulation for 7 d. Representative experiments of a total of 46 conditions comprising 171 array experiments. (b) relative SATB1 mRNA expression in T_{reg} and T_{conv} assessed by qRT-PCR (mean +/- SD, n= 5; * p< 0.05). (c) Western blot analysis of SATB1 protein expression in a representative donor (left) and relative expression of SATB1 (n= 6, mean +/- SD, right; * p< 0.05). (d) flow cytometric analysis of SATB1 protein expression in T_{conv}, and T_{reg} for one representative donor (left) and mean SATB1 expression +/- SD (right, n= 11; * p< 0.05). (e) flow cytometric analysis of SATB1 protein expression in T_{conv}, and T_{reg} after stimulation (3 d; n= 4; * p< 0.05). (f)-(h) for 5 d naïve human T cells were left unstimulated (Tᵤₙₛₜ), stimulated with CD3 and CD28 beads (Tₛₜᵢₘ) or stimulated in the presence of TGFβ to become induced regulatory T cells (iT_{reg}). (f) SATB1 mRNA expression (mean +/- SD) as assessed by qRT-PCR after 5 d (n= 6; * p< 0.05). (g) flow cytometric analysis of SATB1 protein expression after 5 d in one representative experiment (left) and relative expression of SATB1 (right, n= 3, mean +/- SD; * p< 0.05). (h) CBA assessment of IL4 and IFN-γ cytokine secretion (mean +/- SD; * p< 0.05). (i) Analysis of SATB1 mRNA expression (mean +/- SD; * p< 0.05) in T_{conv} and T_{reg} derived from male DEREG mice as well as FOXP3-deficient DEREG x scurfy mice as assessed by qRT-PCR. A representative of two independent experiments is shown. (i) Immunofluorescence staining for SATB1 (red) and FOXP3 (green) protein expression in GFP⁺ T_{reg} sorted from the spleen of female DEREG mice heterozygous for the scurfy mutation counterstained with DAPI (blue). (k) flow cytometric analysis of intracellular SATB1 protein expression in CD4⁺ single positive thymocytes (left, light grey) as well as thymic single positive FOXP3-sufficient (FOXP3⁺, middle, dark grey) and -deficient (FOXP3⁻, right, black) GFP⁺ T_{reg} from female DEREG mice heterozygous for the scurfy mutation shown for one representative donor (left) and as mean SATB1 expression +/- SD (right, n= 2). Isotype control shown as solid line.

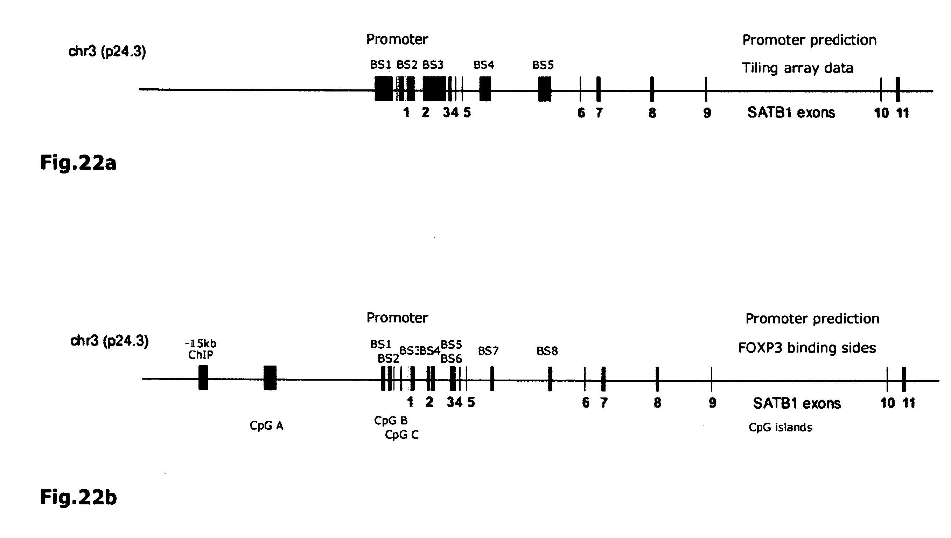

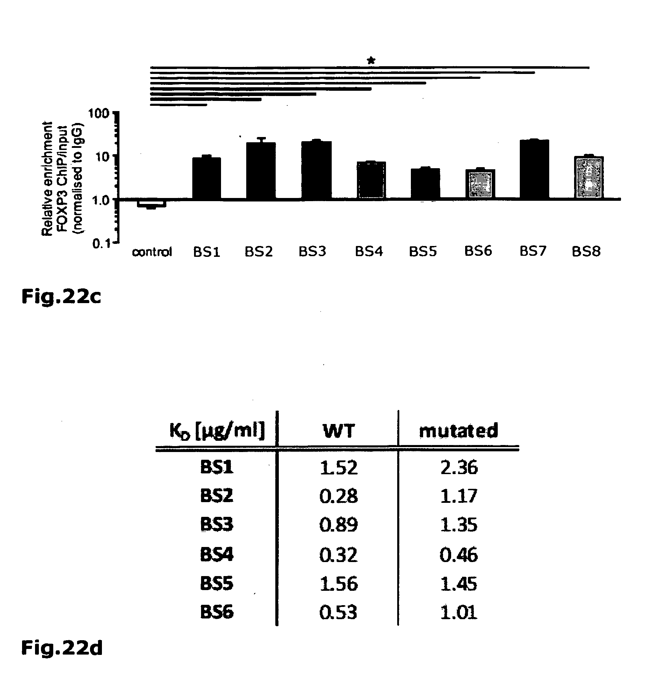

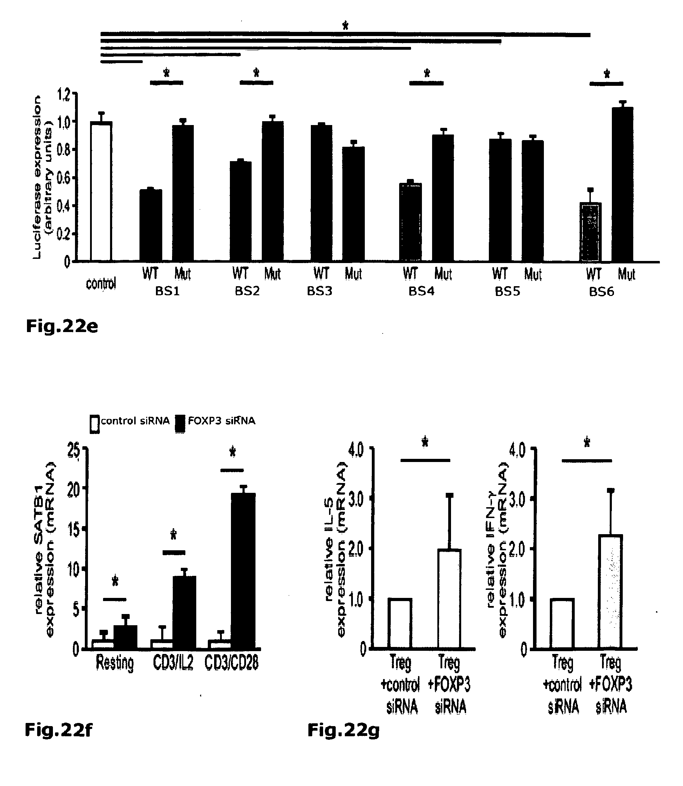

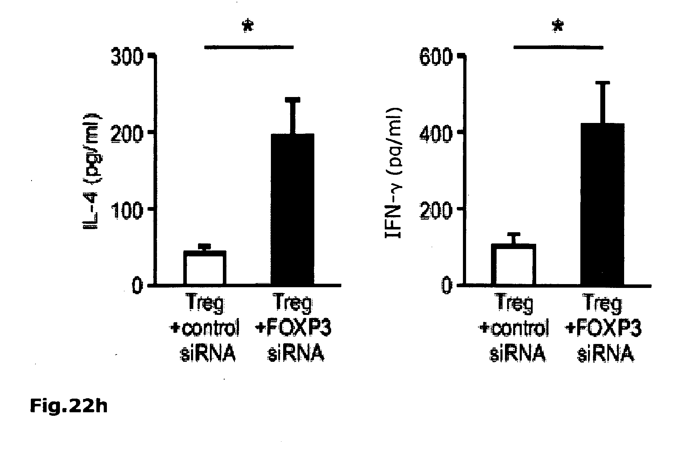

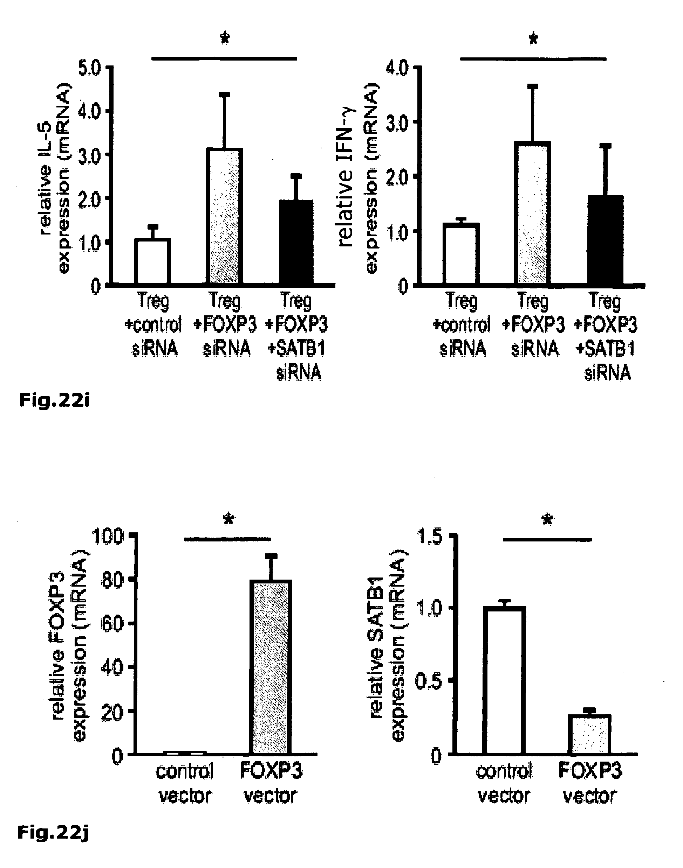

Fig. 22: Direct suppression of SATB1 mRNA transcription by FOXP3. (a) FOXP3. ChIP tiling array data from human expanded T_{reg} cells overlayed to the human SATB1 locus. (b) Representation of the human genomic SATB1 genomic region and the FOXP3 binding sites. (c) FOXP3 binding to the genomic SATB1 regions was assessed by ChIP-qPCR on chromatin isolated from expanded human natural T_{reg}. PCR was performed using a primer set specific for the corresponding region in the SATB1 locus. Enrichment in FOXP3-ChIP over input DNA normalized to control IgG is depicted. Shown here is one representative experiment of 2. (d) K_{D} for FOXP3 binding to the SATB1 locus were defined by RIA for exemplary binding motives. (e) Luciferase activity was assessed by luminometric analysis after transfection of a reporter construct containing the potential FOXP3 binding sites in the genomic SATB1 locus or with a mutated motive. FOXP3 binding was assessed in comparison between cells transfected with a control or FOXP3-expressing vector (mean +/- SD; * p< 0.05) in comparison to the mutated motive. A representative of three independent experiments is shown. (f)-(i) MACS-purified human natural T_{reg} were either transfected with a scrambled control siRNA or FOXP3-specific siRNA, (f) SATB1 mRNA expression (mean +/- SD; n= 6, * p< 0.05) assessed by qRT-PCR in FOXP3-sufficient (control siRNA) and -deficient (FOXP3 siRNA) primary human natural T_{reg} cultivated for 36 h in the presence of CD3 and IL-2 or CD3 and CD28. (g) Analysis of IL-5 and IFN-γ mRNA expression by qRT-PCR (n= 4, mean +/- SD, * p< 0.05) in FOXP3-sufficient (control siRNA) and -deficient (FOXP3 siRNA) primary human natural T_{reg} stimulated for 48 h in the presence CD3 and IL-2 post knockdown. (h) CBA assessment of IL-4 and IFN-γ cytokine secretion of FOXP3-sufficient (control siRNA) and -deficient (FOXP3 siRNA) primary human natural T_{reg} (mean +/- SD, * p< 0.05) stimulated for 48 h in the presence CD3 and IL-2 post knockdown. (i) Assessment of IL-5 and IFN-γ mRNA expression (mean +/- SD) in primary human natural T_{reg} transfected with SATB1-specific siRNA after silencing of FOXP3 48 h post knockdown stimulated with CD3 and CD28 (n= 4, mean +/- SD, one-way ANOVA, * p< 0.05). (i) Analysis of FOXP3 (left) and SATB1 (right) expression in human conventional T_{reg}-depleted CD4⁺ T cells lentivirally transfected with FOPX3 by qRT-PCR (mean +/- SD, n= 5, * p< 0.05).

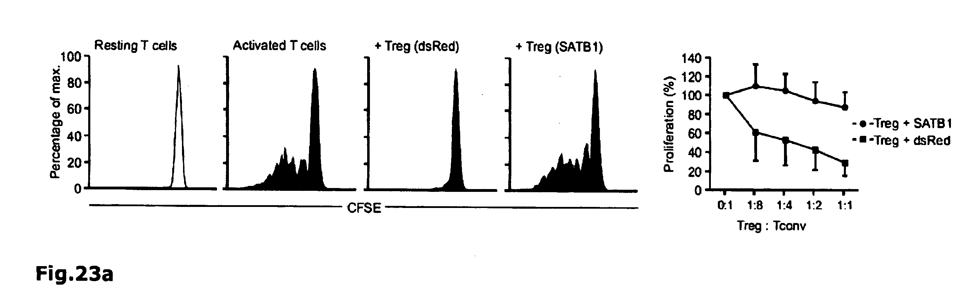

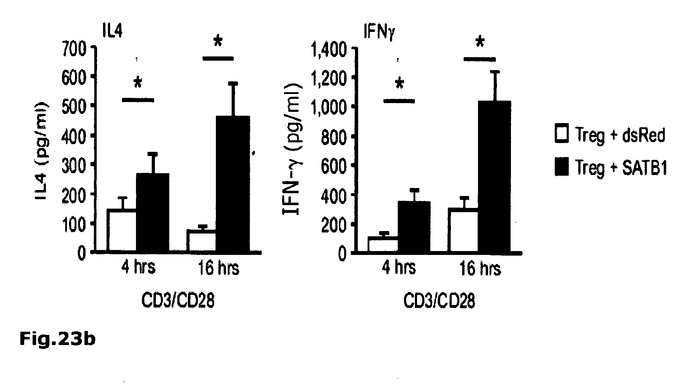

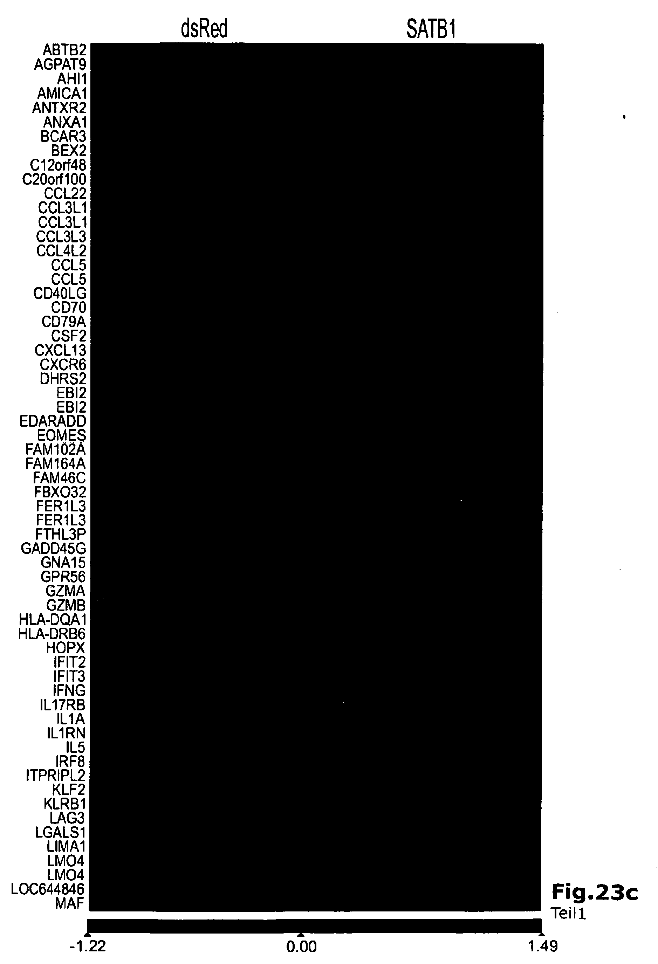

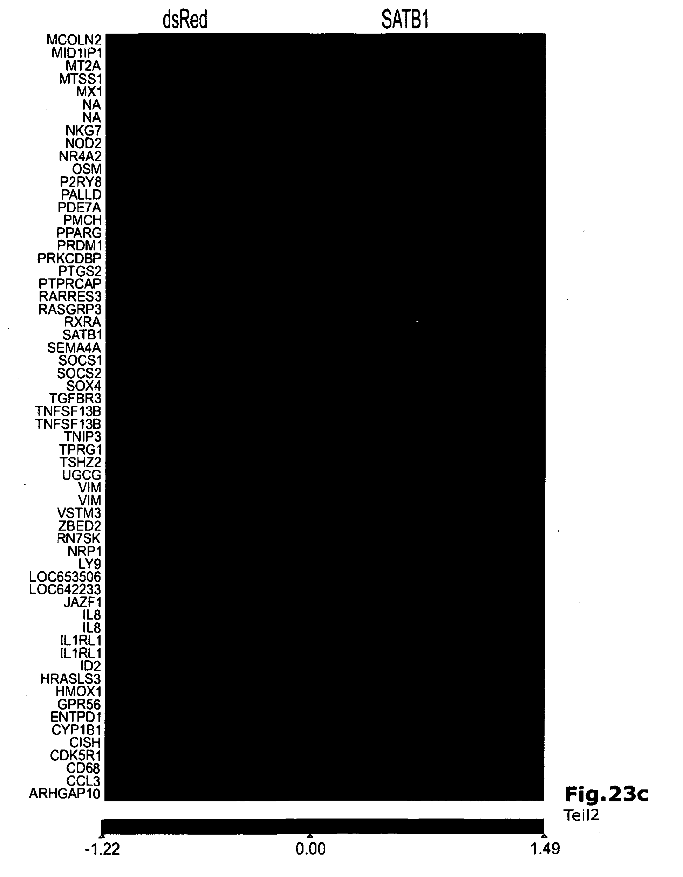

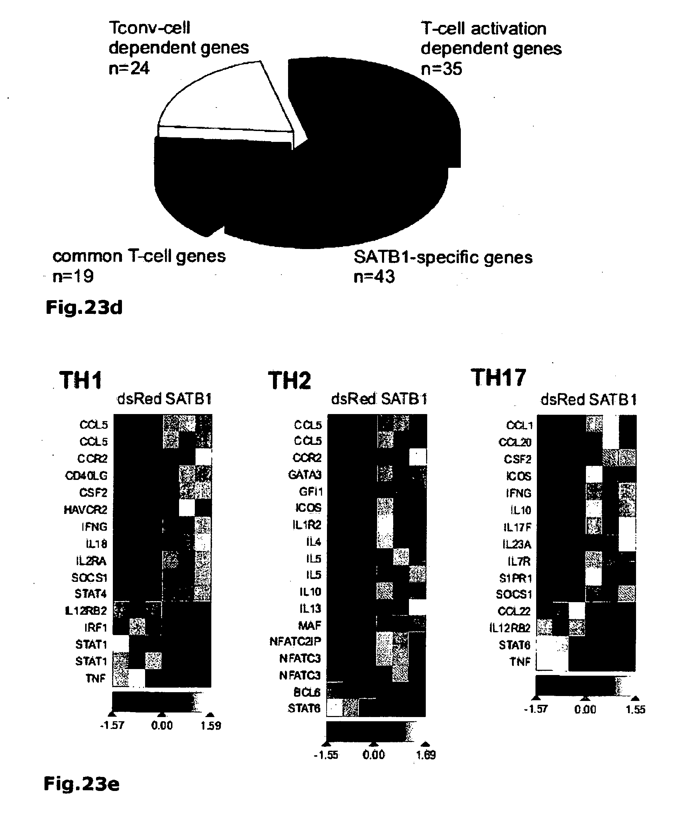

Fig. 23: SATB1 expression reprograms regulatory T cells into effector T cells. (a) Analysis of suppressive function of human T_{reg} lentivirally transfected with SATB1 (right, blue) or control vector (dsRed, left, red) shown for one representative donor (left) and as mean proliferation of CD8⁺ T cells +/- SD (right, n= 3, * p< 0.05). (b) CBA assessment of IL-4 and IFN-γ cytokine secretion of SATB1-transduced as well as control-transduced T_{reg} 4 and 16 h after stimulation with CD3/CD28-coated beads (mean +/- SD, * p<0.05). (c) Up- and down-regulated genes in SATB1-transduced T_{reg}. Data were z-score normalized. (d) Classification of SATB1-induced genes according to the comparisons between T_{conv} and T_{reg} cells, unstimulated and CD3/CD28-stimulated naive T cells and common genes to both subsets (T_{conv} and activated T cells). (e) Expression pattern of SATB1-dependent genes potentially contributing to reprogramming of T_{reg} into T_{effector}, classified to TH1, TH2, and TH17 - specific genes. Data were z-score normalized.

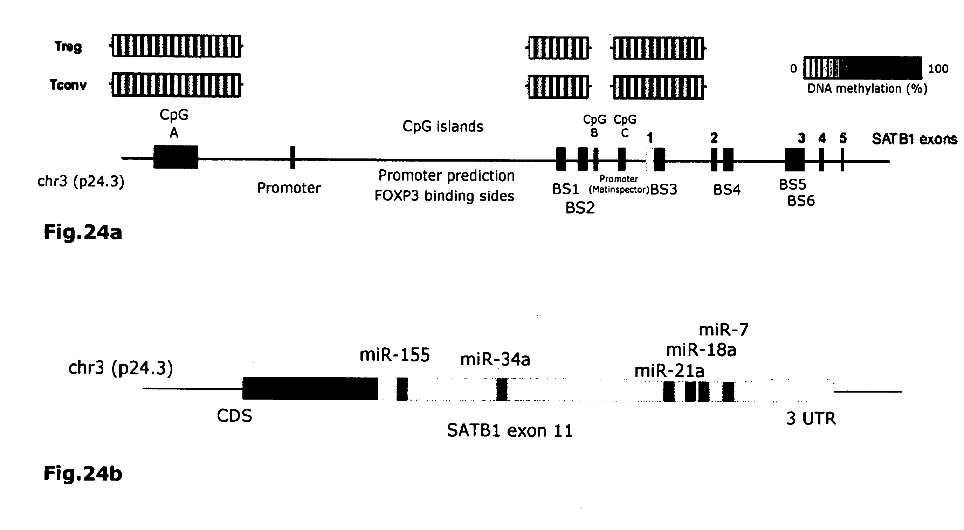

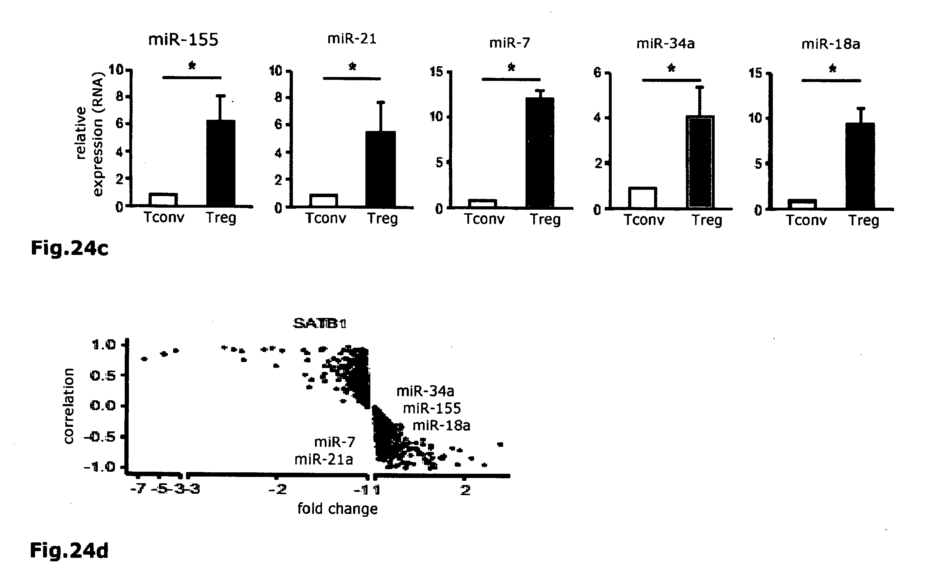

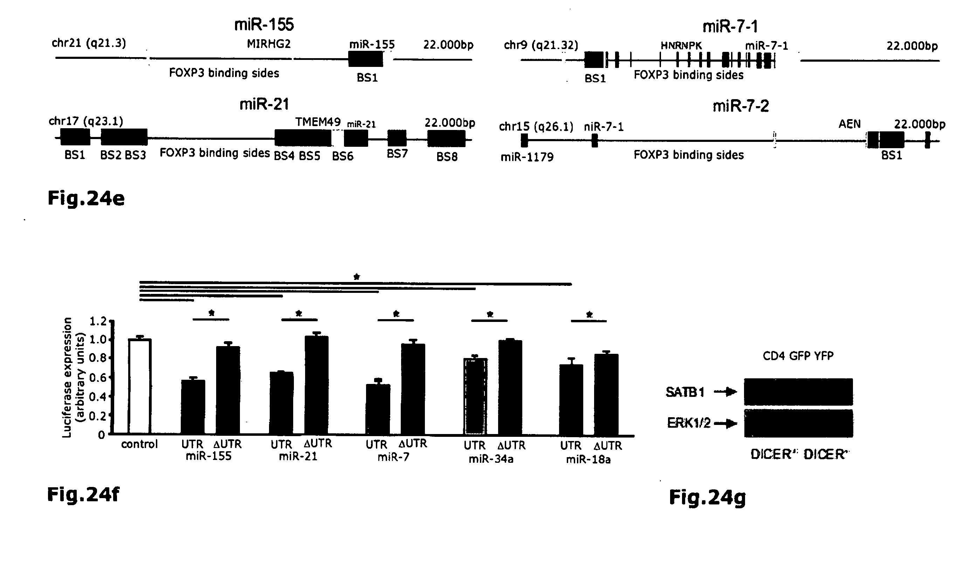

Fig. 24: Regulation of SATB1 by miRNA. (a) DNA methylation of the predicted CpG-islands in the genomic region of SATB1 in T_{reg} and T_{conv}. (b) Representation of the human genomic SATB1 3' UTR and the conserved miRNA binding sites. (c) Mean miRNA expression for miR-155, miR-21, miR-7, miR-34a, and miR-18a in human natural T_{reg} in comparison to T_{conv} (mean +/- SD; n= 5, * p< 0.05) as assessed by qPCR. (d) Correlation of miRNA expression with SATB1 mRNA expression is plotted against miRNA fold change (T_{reg} vs. T_{conv}) for all 735 miRNA assessed. Highlighted are miR-155, miR-21, miR-7, miR-34a, and miR-18a. (e) FOXP3-binding to the genomic locus of miR-155, miR-21, and miR-7 in human natural T_{reg} cells as defined byFOXP3 ChIP tiling arrays. (f) Luciferase activity was assessed by luminometric analysis after transfection of a reporter construct containing the SATB1 3' UTR. Regulation of SATB1 expression by miRNA's was assessed by transfection of the corresponding miRNA in comparison with a control miRNA (mean +/- SD; * p< 0.05). Mutation of the miRNA motif was used to demonstrate specificity. A representative of three independent experiments is shown. (g) Western blot analysis of SATB1 protein expression in sorted T_{reg} from mice with a T_{reg}-specific complete DICER loss (DICER^{fl/fl}) in comparison to DICER^{wt/fl} T_{reg}.

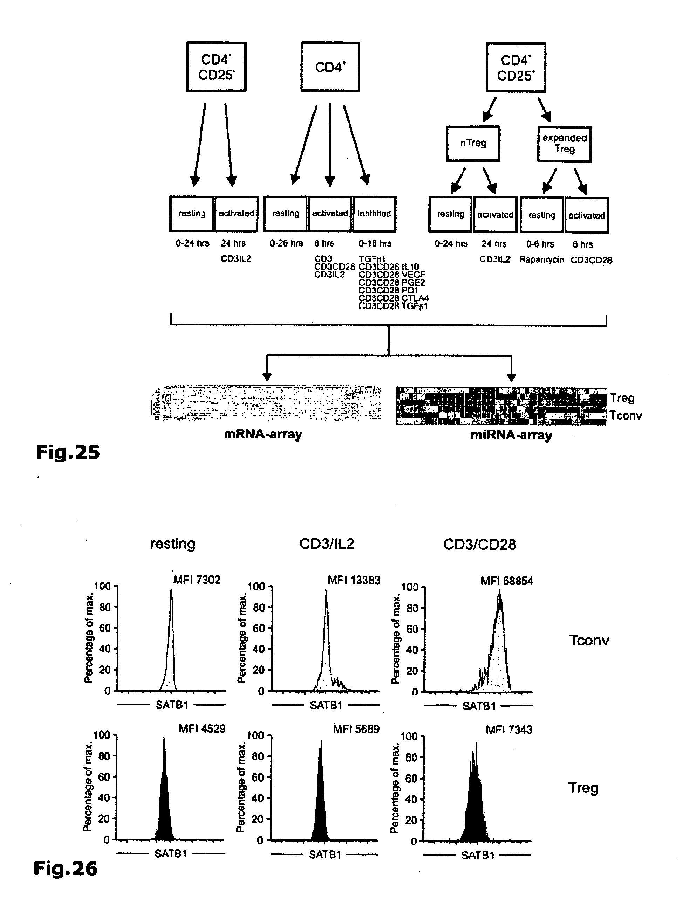

Fig. 25: Layout of microarray experiments performed to identify SATB1 expression and microRNA regulation in T_{reg}. Human CD4⁺ T cells, CD4⁺ CD25⁻ T_{conv}, CD4⁺ CD25⁺ T_{reg}, and expanded T_{reg} were assessed either directly after isolation (resting), after up to 24 h of cell culture without further stimulation (resting), or after activation by various stimuli (activated). Included are also inhibitory conditions of CD4⁺ T cells stimulated in the presence of inhibitory signals including IL-10, prostaglandin-E2 (PGE2), PD1, CTLA-4 or TGFβ1. If not otherwise indicated, cells were stimulated for 8 h prior harvesting for microarray analysis (see also Table 1).
Figure 26: Flow cytometric assessment of SATB1 protein expression in stimulated T_{conv} and T_{reg}. Flow cytometric analysis of SATB1 protein expression in T_{conv} and T_{reg} after stimulation with CD3 and IL-2 or CD3 and CD28 for 3 d exemplified for one donor. MFI values are presented in the upper right corner for T_{reg} and T_{conv} respectively.

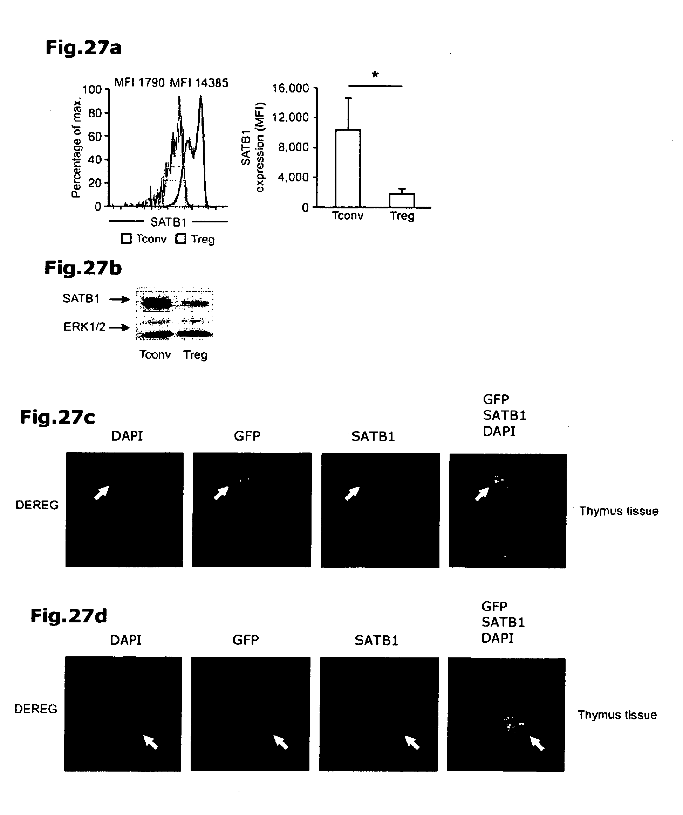

Fig. 27: Analysis of SATB1 expression in murine T_{reg}. (a) Thymic T_{reg} as well as T_{conv} from DEREG mice were stained for CD4, CD8, FOXP3, and SATB1 and gated on CD4, CD8, GFP, and FOXP3 expression. SATB1 expression in T_{reg} and T_{conv} cells from thymus tissue was assessed by flow cytometry and is shown for one representative donor (left) and as mean SATB1 expression +/- SD (right, n=3). SATB1 expression in CD4⁺ single-positive thymocytes was considerably higher than in CD4⁺ T_{conv} from the spleen (data not shown) as SATB1 expression is essential for thymocyte development (Alvarez, J. D. et al., Genes Dev 14, 521-535 (2000)), yet a significant downregulation of SATB1 in T_{reg} was detectable in both tissues (data not shown). MFI values are presented in the upper left or right corner for T_{reg} and T_{conv} respectively. (b) Western blot analysis of SATB1 protein expression in murine T_{reg} and T_{conv}. (c) and (d) Immunofluorescence staining for SATB1 (red) and GFP (green) protein expression in thymocytes from male DEREG mice counterstained with DAPI (blue).

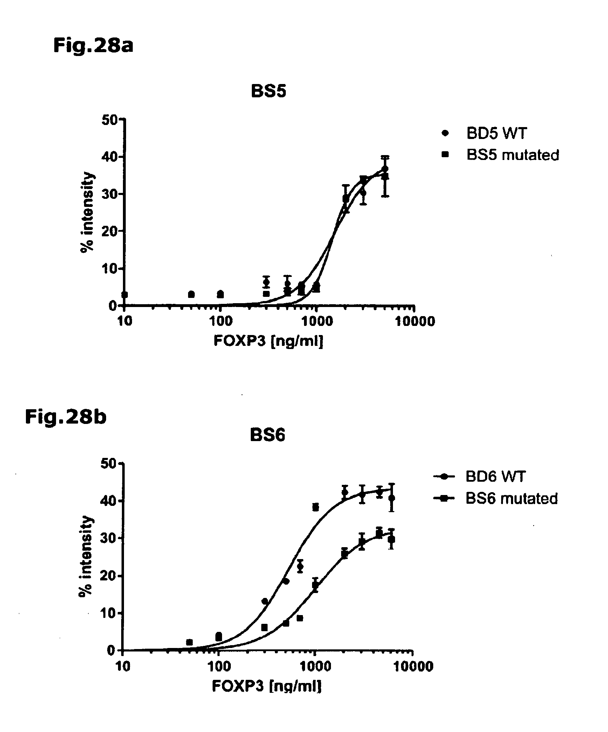

Fig. 28: Assessment of FOXP3 binding to the SATB1 locus. KD for FOXP3 binding to the SATB1 locus were defined by RIA for the identified FOXP3 binding motives in the SATB1 genomic locus in comparison to mutated motifs as exemplified for BS5 and BS6.

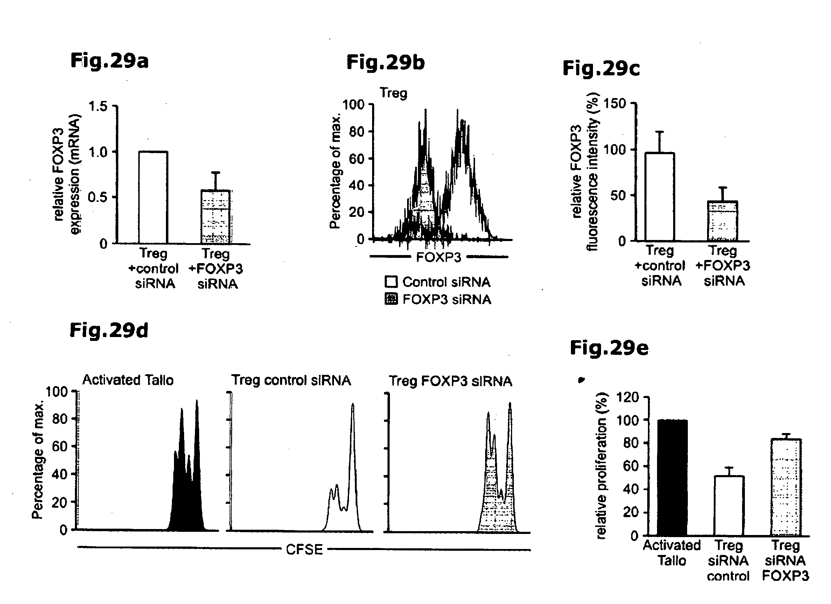

Fig. 29: Knockdown of FOXP3 in primary human T_{reg}. Human T_{reg} were either transfected with control siRNA or FOXP3-specific siRNA and assessed 48 h post knockdown. (a) relative FOXP3 mRNA expression (mean +/- SD, n=6, * p<0.05). (b) Representative flow cytometric analysis of intracellular FOXP3 expression 48 h post FOXP3 knockdown in T_{reg}. (c) Mean FOXP3 protein expression (mean +/- SD, n= 6, * p< 0.05). (d) Suppressive function of control or FOXP3 siRNA treated T_{reg} assessed in a standard suppressive assay using CD4⁺ allogeneic T cells as readout. One representative experiment is shown. (e) Mean inhibitory capacity (mean +/- SD, n= 3, * p< 0.05).

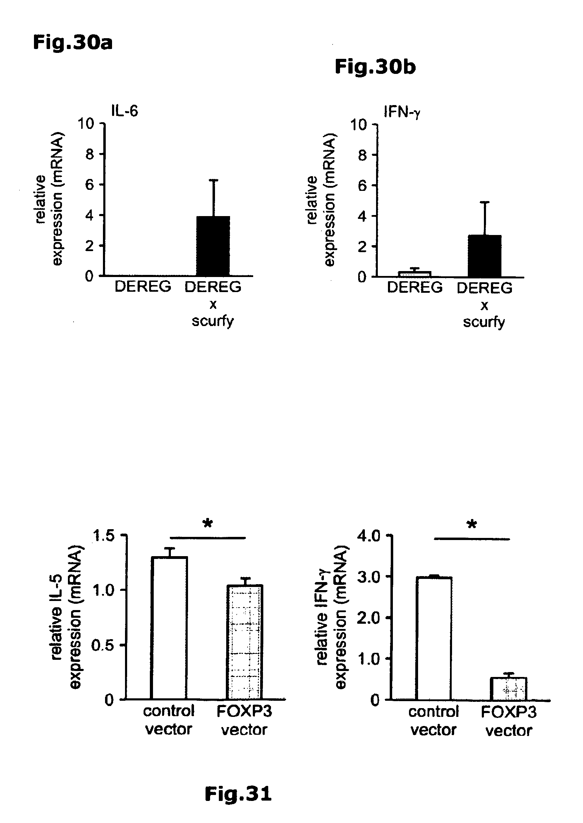

Fig. 30: TH1/TH2 differentiation of T_{reg} from DEREG x scurfy mice. To assess whether T_{reg} expressing SATB1 differentiate in T-helper cells expressing TH1/TH2 cytokines, we isolated GFP⁺ T_{reg} and analyzed (a) IL-6 and (b) IFN-γ mRNA production by T_{reg} derived from DEREG or DEREG x scurfy mice. A representative of two independent experiments is shown.
Fig. 31: T_{conv} transfected with FOXP3 show reduced cytokine production. Analysis of IL-5 (left) and IFN-γ (right) expression in human conventional T_{reg}-depleted CD4⁺ T cells lentivirally transduced with FOPX3 by qRT-PCR (mean +/- SD, n= 5, * p< 0.05).

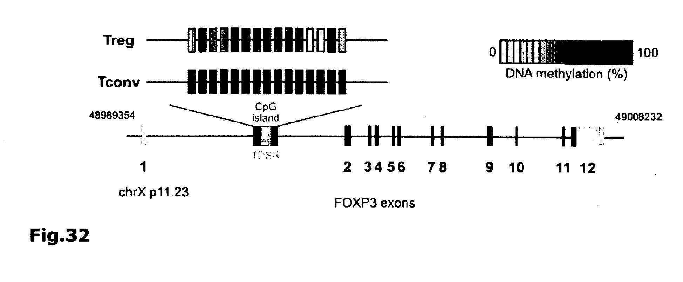

Fig. 32: DNA methylation of the CpG island of the FOXP3 locus in T_{reg} and T_{conv}.

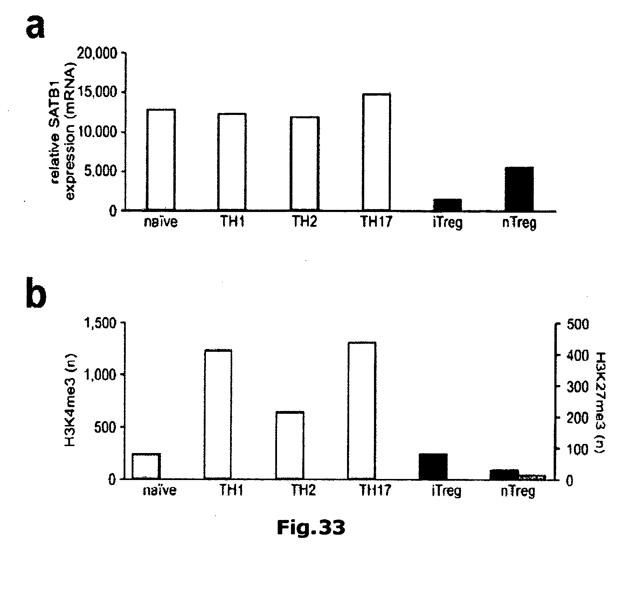

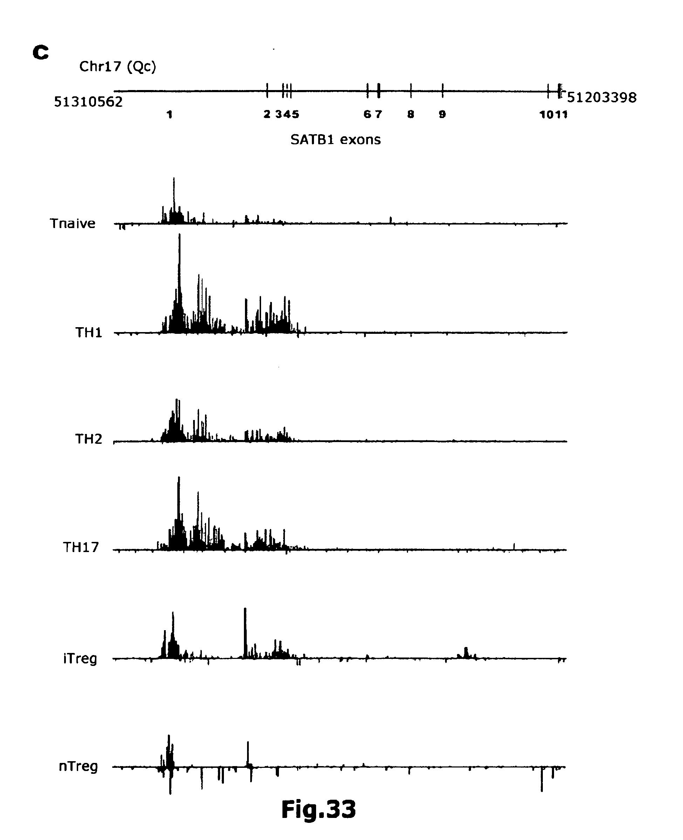

Fig. 33: Histone methylation at the SATB1 gene locus. Very recently published data on genome-wide histone methylation (Wei, G. et al., Immunity 30, 155-167, (2009)) were reanalyzed for SATB1 expression and histone methylation maps in murine naive T cells, T_{effector} (TH1, TH2, resp. TH17), iT_{reg} and nT_{reg}. (a) expression of SATB1 as assessed by microarray analysis. (b) and (c) ChIP-sequencing data were re-analyzed for the SATB1 locus. Trimethylation of H3K4 is associated with gene activation, whereas di- and trimethylation of H3K27 are associated with gene repression. Low to absent trimethylation of H3K27 was detected in the T-cell subsets analyzed, while T_{effector} showed high levels of H3K4 methylation and T_{reg} lower methylation. (b) cumulative data for Tnaive, TH1, TH2, TH17. iT_{reg}, and nT_{reg}. (c) analysis of trimethylation islands (red: H3K4, blue: H3K27) mapped on the genomic SATB1 locus.

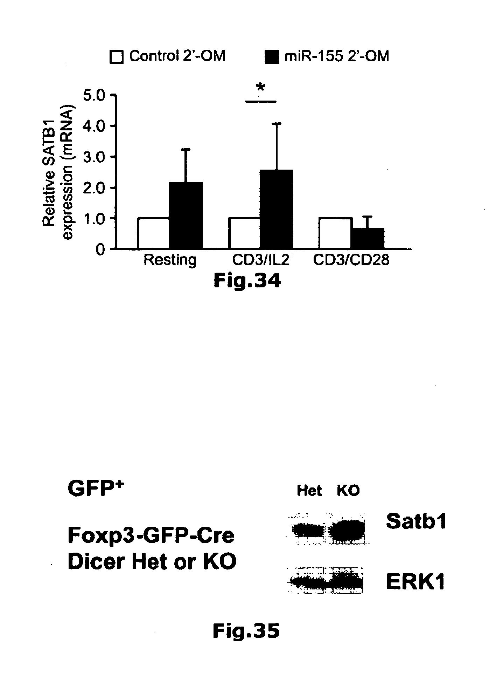

Fig. 34: SATB1 expression after siRNA-mediated silencing of miR-155 in T_{reg}. MACS-purified human natural T_{reg} were transfected with either a control or miR-155 inhibitor and regulation of SATB1 mRNA expression was assessed 48 h post knockdown by qRT-PCR (mean +/- SD, n= 5, * p< 0.05) in unstimulated, CD3 and IL-2, or CD3 and CD28 stimulated T_{reg}.
Fig. 35: SATB1 expression in miRNA-depleted T_{reg}. Western blot analysis of SATB1 protein expression in sorted T_{reg} from mice with a T_{reg}-specific complete DICER loss (DICER^{fl/fl}) in comparison to DICER^{wt/fl} T_{reg}.

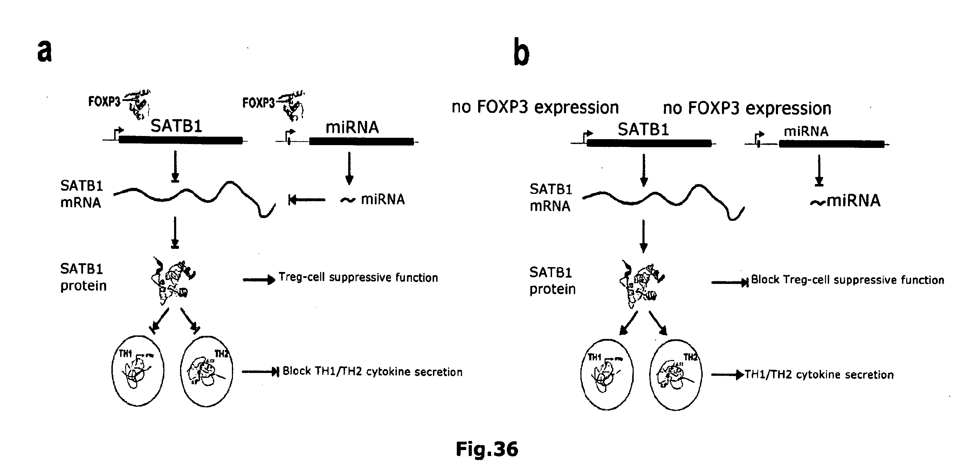

Fig. 36: Model for the mode of action of FOXP3. (a), (b) Model for the FOXP3- and miRNA-mediated SATB1-dependent remodelling of the respective genomic loci for the release of TH1 and TH2 cytokines and the induction of suppressive function of T_{reg}.

### Detailed Description of the Invention

The methods of aspects (1) to (5) of the invention identify the regulatory T cells in the cell population due to the significant reduction (or even absence) of binding of such regulatory T cells to ligands that specifically bind to SATB1 as compared to the remaining cells of the cell population. In other words, all cells (except for the regulatory T cells) of the cell population show binding with said ligands.

"Ligands" according to the invention can be antibodies or fragments thereof, including human, murine, rabbit and goat antibodies and antibody fragments. Particularly suitable ligands are monoclonal antibodies or fragments thereof.

According to the the ligands/antibodies carry functional moieties allowing detection, including but not limited to labels (such as fluorescence and bioluminescence dyes and radioactive labels), ligands (such as DNA, RNA and protein molecules, Ig fusion molecules, bifunctional RNA molecules and cell membrane penetrating molecules that are coupled to a ligand), toxins (such as ricine, lectine and diphtheriatoxin).

The method of the invention is applicable to any type of cell population including, but not limited to, cell culture, whole blood and fractions of whole blood, and cells of any origin including, but not limited to, mammalian cells such as human cells and murine cells.

In a particularly preferred embodiment the method is suitable for quality control of T cell populations, notably of regulatory T cell populations, where contaminating effector T cells are detected in the population of regulatory T cells, or an effector T cell population, where contaminating regulatory T cells are detected in the population of effector T cells.

The method of the invention may be combined with other detection methods for regulatory T cells known in the art. For identifying human T cells it is desirable that the T cell population is contacted with one or more ligands that specifically bind to CD4, CD25 and/or CD127 on the T cells. A further method is assaying for FOXP3 expression. The kit of aspect (6) of the invention may - apart from the ligands/antibodies/antibody fragments - comprise buffers and reagents for performing the detection method of the invention, standard cell suspensions and also reagents for performing the additional detection methods referred to above.

The invention is furthermore described in the following examples which are, however, not to be construed as a limitation of the invention.

### Examples

### Materials and Methods

Mice: C57BL/6 (B6) mice were purchased from the Jackson Laboratory. *DEREG, scurfy* and *DEREG x scurfy* mice were previously described (Brunkow, M.E. et al., Nat. Genet. 27:68-73 (2001); Lahl, K. et al., J. Immunol. in revision; Lahl K. et al., J. Exp. Med. 204:57-63 (2007)). The male *DEREG x scurfy* mice were indistinguishable from *scurfy* mice in regard to the immunological and clinical manifestations of autoimmunity while female *DEREG* mice heterozygous for FOXP3 were symptom free. Mice were housed under specific pathogen-free conditions and used according to the guidelines of the Institutional Animal Care Committee at the Institute for Medical Microbiology, Immunology and Hygiene, TU Munich.

Antibodies and FACS analysis: Fluorescent-dye-conjugated antibodies were purchased from BD, Biolegend, or eBioscience. Alexa 647-conjugated mouse anti-human SATB1 monoclonal antibody (clone 14) cross-reactive to murine SATB1 was prepared by labeling the commercially available antibody (BD Biosciences material number 611182) with the dye. FACS data were acquired on a FACSCanto flow cytometer (Becton Dickinson) and analyzed using FlowJo software package (Tri-Star). Intracellular staining of human and murine FOXP3 and SATB1 was conducted using either the human or mouse FOXP3 Mouse Regulatory T cell Staining Kit (Biolegend) with the addition of FcR-blocking reagents (CD16/CD32 or human IgG) 15 min before intranuclear staining.

Purification and sorting of human T_{reg}: Human T_{reg} and T_{effector} were purified from whole blood of healthy human donors in compliance with institutional review board (IRB) protocols by negative selection using CD4-RosetteSep (Stem Cell), followed by positive-selection using CD25-specific MACS beads (Miltenyi Biotech) or sorting on a FACSDiVa cell sorter (Becton Dickinson) after incubating cells with combinations of fluorochrome-labeled monoclonal antibodies to CD4, CD25, and CD127. For experiments with non-sorted cells, only samples with >95% T_{reg} were used.

Purification and sorting of murine T_{reg}: Murine GFP⁺ T_{reg} were purified from thymus, spleen, or peripheral lymph nodes by sorting on a MoFlo high performance cytometer (Beckman Coulter) directly or after positive enrichment of CD4⁺ T cells after positive-selection using CD4-specific MACS beads (Miltenyi Biotech).

Generation of induced T_{reg}: Human CD4⁺ lymphocytes were purified from whole blood of healthy human donors by negative selection using CD4-RosetteSep (Stem Cell). This population was then incubated with CD25-specific MACS beads (Miltenyi Biotech). After negative selection, conventional CD4⁺ lymphocytes were incubated with CD45RA-specific MACS beads (Miltenyi Biotech). Naïve conventional T cells were obtained by passing the cell mixture over MidiMACS magnetic separation columns (Miltenyi Biotech) and collecting the CD4⁺ CD25⁻ CD45RA⁺ T cells. Naive T_{reg}-depleted CD4⁺ T cells (5 x 10⁴ cells well⁻¹) were stimulated in serum-free Aim-V/X-Cell (50%/50% V/V) medium with 5 x 10⁴ magnetic beads coated with 5% CD3 (OKT3, Ortho Biotech), 12% CD28 (9.3), and 83% anti-MHC-I (W6/32) monoclonal antibody well⁻¹ and TGFß1 (R&D systems) 5 ng ml⁻¹ for a period of 7 days in the absence of IL-2. The TGFß1 was not acid-treated before addition. The described composition of beads was optimized for the induction of T_{reg} cells.

*In vitro* suppression assay: For *in vitro* suppression assays, CFSE-labeled T_{effector} (1 x 10⁵ cells well⁻¹) were co-cultured with PKH-26-labeled natural or induced T_{reg} at indicated ratios in the presence of CD3/CD28/MHC-I-coated magnetic beads (3.3 x 10⁴ beads well⁻¹) in 96-well plates in X-Vivo-15 medium supplemented with 10% FCS for 72 h. CFSE dilution was measured on a FACSCanto flow cytometer.

Cytokine cytometric bead array: IL-4, IL-6, and IFN-gamma concentrations were measured using the human TH1/TH2 cytokine kit II (BD Pharmingen).

qRT-PCR on human samples: Total RNA from T_{conv} or T_{reg} was used to generate cDNA along with the Transcriptor First Strand cDNA synthesis kit (Roche Diagnostics). qRT-PCR was performed using the LightCycler Taqman master kit and the Universal Probe Library assay specific for SATB1, FOXP3, IL-5, IFN-gamma and beta-2 microglobulin (B2M; Roche Diagnostics). For each experiment at least two technical replicates were performed. Results were normalized to B2M expression.

Western blot analysis: Cell lysates from purified T_{conv}, iT_{reg}, and nT_{reg} were prepared as previously described (Classen, S. et al., J. Immunol. 178:6931-6940 (2007)) followed by western blotting with SATB1 or beta-actin antibodies.

Whole-genome gene expression in human cells: All RNA was extracted using TRIZOL (Invitrogen) and purified in our laboratory using standard methods. Sample amplification, labeling and hybridization on Illumina WG6 Sentrix BeadChips V1 were performed for all arrays in this study according to the manufacturer's instructions (Illumina) using an Illumina BeadStation. All data analyses were performed by using Bioconductor for the statistical software R (http://www.r-project.org). Expression values were normalized and summarized by using the IIIuminaGUI package. From the resulting data sets we extracted a list of genes with a significant different expression in T_{reg} compared to T_{conv}. Microarray data can be accessed under GSE15390.

Immunofluorescence microscopy: Unpurified lymphocytes from male *DEREG* or *DEREG x scurfy* mice or GFP⁺ T_{reg} from female heterozygous *DEREG x scurfy* mice purified from thymus, lymph nodes and spleens were fixed in cold paraformaldehyde for 10 min, washed with PBS, permeabilized with Triton-X and pre-blocked in PBS containing 10% normal goat serum and 1% gelatin from cold water fish skin for 30 min. Slides were then incubated in combinations of primary antibodies (rabbit anti-GFP, mouse anti-FOXP3, rat anti-CD4, mouse anti-SATB1-AF647) for 60 min, washed twice, and incubated with secondary antibodies (anti-rabbit-AF488, anti-mouse-AF555, anti-rat-AF555) for 60 min, stained with DAPI and fluorescence was examined using a Olympus Fluoview FV1000 confocal microscope.

qRT-PCR of murine T_{reg}: Total RNA was extracted with TRIZOL reagent from FOXP3-sufficient and -deficient CD4⁺ GFP⁺ T_{reg} as well as T_{conv} FACS-purified from male *DEREG* and *DEREG x scurfy* mice, respectively. Complementary DNA was synthesized (Miltenyi). qPCR was performed using the LightCycler Taqman master kit and the Universal Probe Library assay (Roche Diagnostics). PCR primer sequences are listed in Table 2.

Electromobility shift assays, chromatin immunoprecipitation and qPCR: EMSA were performed with fluorescent-dye conjugated oligonucleotides as described previously (Mantel, P.Y. et al., J. Immunol. 176:3593-3602 (2006)) with nuclear extracts from expanded human MACS purified CD4⁺ CD25⁺ T_{reg} according to the manufacturer's recommendations (LI-COR) and analyzed with the Odyssey infrared imaging system following electrophoresis. FOXP3 (eBioscience) and IgG antibody (BD Bioscience) ChIPs were performed using expanded human MACS purified CD4⁺CD25⁺ T_{reg} following the manufacturer's instructions (Active Motif). Relative abundance of regions of interest in precipitated DNA was measured by semi-quantitative PCR. Additionally, qPCRs were performed using iQ SYBR Green Supermix (Bio-Rad) with equal results. Oligonucleotide and PCR primer sequences are listed in Table 3.

miRNA profiling and aRT-PCR: All RNA was extracted using TRIZOL (Invitrogen) and purified in our laboratory using standard methods. Sample amplification, labeling and hybridization on Illumina miRNA array matrix were performed with the human v1 MicroRNA Expression Profiling kit for all arrays in this study according to the manufacturer's instructions (Illumina) using an Illumina BeadStation. All data analyses were performed by using Bioconductor for the statistical software R (http://www.r-project.org). Expression values were normalized and summarized by using the IlluminaGUI package. From the resulting data sets we extracted a list of miRNAs with a significant different expression in T_{reg} compared to T_{conv}. For miRNA-specific qRT-PCR, total RNA was extracted using TRIZOL. First strand complementary DNA for each miRNA assessed was synthesized by using the TaqMan MicroRNA RT kit and the corresponding miRNA specific kit (Apllied Biosystems). Levels of miRNA were measured by qPCR using the TaqMan Universal PCR MasterMix (Applied Biosystems) on an iQ5 Cycler (Bio-Rad). Ubiquitously expressed U6 small nuclear RNA or miR-26b were used for normalization. PCR primer sequences are listed in Table 4.

Gene-specific mRNA silencing, miRNA knockdown and agonistic miRNA: All siRNAs as well as the miRNA mimics and inhibitors were purchased from Biomers or Dharmacon. miRNA mimics were designed according to the sequences published in miRBase and resembling the double-stranded Dicer-cleavage products. miRNA-inhibitors were designed as single-stranded antisense 2'OM oligonucelotides. These were transfected into freshly isolated primary human T_{reg} with nucleofection as previously described (Mantei, A. et al., Eur. J. Immunol. 38:2616-2625 (2008)). For luciferase assays, HEK293T cells were transfected with both the reporter plasmids and the small RNA duplexes using Lipofectamine 2000 in a 96-well format and luciferase activity was measured 24 h later.

Luciferase assays: Human embryonic kidney (HEK) 293T (ATCC CRL-11268) were maintained in DMEM containing 10% heat-inactivated fetal calf serum and penicillin/streptomycin. The 200 bp surrounding the human FOXP3 binding site in intron 2 of SATB1 and the 3'UTR of human SATB1 was amplified using PCR and cloned into a psiCHECK II vector to generate psiCHECK II-SATB1-intron 2 respectively psiCHECK II-SATB1-3'UTR. These constructs (2 ng) were co-transfected seperately into HEK293T cells in 96-well plates together with 2 ng of control plasmid or plasmids expressing FOXP3 respectively a miRNA mimic for miR-155 or a scrambled control miRNA. Lysis and analysis were performed 24 h post transfection using the Promega Dual Luciferase Kit. Luciferase activity was counted in a Mithras plate reader (Berthold).

FOXP3 transductions: T_{reg}-depleted human CD4⁺ T_{conv} cells were lentivirally transduced with a pELNS YFP 2A FOXP3 or control plasmids containing GFP as previously described (Basu, S. et al., J. Immunol., 180:5794-5798 (2008)) and assessed after 72-120 h for SATB1 expression.

Bisulphite sequencing: Genomic DNA from human T_{reg} cells and conventional T cells purified by negative selection using CD4-RosetteSep (Stem Cell), followed by sorting on a FACSDiVa cell sorter (Becton Dickinson) after incubating cells with combinations of fluorochrome-labeled monoclonal antibodies to CD4, CD25, and CD127 was isolated using the phenol/chloroform extraction following the supplier's recommendations. Sodium bisulphate treatment of genomic DNA was performed resulting in the deamination of unmethylated cytosines to uracil, whereas methylated cytosines remain unchanged. After amplification PCR products were purified and sequenced in both directions.

Statistical analysis: Mann-Whitney tests and student's t-tests were performed with SPSS 15.0 software.

Generation of antibodies: One method to generate antibodies against SATB1 involves administering an antigen presenting cell (APC) to animals, e.g. mouse, rat, rabbit, goat. This results in the activation of B-cells to produce antibodies recognizing T_{reg} cells in a SATB1 specific fashion. The APC can be pulsed with SATB1 or a peptide of SATB1 that binds to a major histocompatibility complex molecule.

Another method includes the generation of antibodies against SATB1 by administering SATB1 or a peptide of SATB1 that binds to a major histocompatibility complex molecule, which is processed by an antigen presenting cell, which, in turn, activates B-cells to produce antibodies recognizing T_{reg} cells in a SATB1 specific fashion. The SATB1 polypeptide or peptide of SATB1 used in this method can be administered in association with an adjuvant.

Alternatively, one method involves administering a nucleic acid molecule encoding SATB1 or a peptide of SATB1 that binds to a major histocompatibility complex molecule. The nucleic acid molecule is expressed so that it can be processed by an antigen presenting cells, which activate B-cells to produce antibodies recognizing SATB1 in a SATB1 specific fashion. The nucleic acid molecule encoding SATB1 or a peptide of SATB1 can be present in an expression vector.

After an animal has been challenged several times with SATB1 B cells from the spleen or lymph nodes are then fused with myeloma tumor cells that can grow indefinitely in culture and that have lost the ability to produce antibodies. This fusion is done by making the cell membranes more permeable by the use of polyethylene glycol or electroporation. The fused hybridomas cells are sufficiently diluted to ensure clonality and grown. The antibodies from the different clones are then tested for their ability to bind to the antigen (for example with a test such as ELISA) or immuno-dot blot, and the most sensitive one is picked out. Monoclonal antibodies are then produced in cell culture by e.g. fermentation chambers.

Another method of generating antibodies against SATB1 involves usage of SATB1 or a peptide of SATB1 to bind antibodies expressed by a phage library. Numerous antibodies are expressed in the library as fusions with the coat protein of a bacteriophage, so that they are displayed on the surface of the viral particle. DNA extracted from interacting phages contains the sequences of the specific antibodies recognizing SATB1 in a SATB1 specific fashion.

**Table 1: SATB1 expression**

| No | n | Donor celltype | condition |
|---|---|---|---|
| 1 | 4 | healthy | CD4+ 12 h cultured then CD3CD28 activated for 8 h |
| 2 | 4 | healthy | CD4+ 8 h CD3CD28 activated |
| 3 | 4 | healthy | CD4+ 8 h CD3CD28 activated and TGFb1 |
| 4 | 4 | healthy | CD4+ 12 h cultured then CD3CD28 activated and TGFb1 for 8 h |
| 5 | 4 | healthy | CD4+ 8 h CD3CD28 activated and VEGF |
| 6 | 3 | healthy | CD4+ 12 h cultured then CD3CD28 activated and VEGF for 8 h |
| 7 | 4 | healthy | CD4+ 8 h CD3CD28 activated and IL10 |
| 8 | 3 | healthy | CD4+ 12 h cultured then CD3CD28 activated and IL10 for 8 h |
| 9 | 4 | healthy | CD4+ 8 h CD3CD28 activated and PGE2 |
| 10 | 4 | healthy | CD4+ 8 h CD3CD28 activated and PD1 |
| 11 | 4 | healthy | CD4+ 8 h CD3CD28 activated and CTLA4 |
| 12 | 4 | healthy | CD4+ 8 h CD3 activated |
| 13 | 3 | healthy | CD4+ 18 h cultured then TGFb1 for 1 h |
| 14 | 3 | healthy | CD4+ 18 h cultured then TGFb1 for 2 h |
| 15 | 3 | healthy | CD4+ 18 h cultured then TGFb1 for 8 h |
| 16 | 4 | healthy | CD4+ 8 h cultured |
| 17 | 4 | healthy | CD4+ 12 h cultured |
| 18 | 3 | healthy | CD4+ 18 h cultured |
| 19 | 3 | healthy | CD4+ 19 h cultured |
| 20 | 6 | healthy | CD4+ 20 h cultured |
| 21 | 3 | healthy | CD4+ 26 h cultured |
| 22 | 4 | healthy | CD4+ 12 h cultured then 1ng/ml TGFb1 for 8 h |
| 23 | 4 | healthy | CD4+ 12 h cultured then 10ng/ml TGFb1 for 8 h |
| 24 | 3 | healthy | CD4+ 18 h TGFb1 |
| 25 | 7 | healthy | CD4+ untreated |
| 26 | 4 | healthy | CD4+CD25- untreated |
| 27 | 4 | CLL | CD4+CD25- untreated |
| 28 | 2 | healthy | CD4+CD25- untreated |
| 29 | 2 | healthy | CD4+CD25- untreated |
| 30 | 3 | healthy | CD4+CD25- 6 h cultured |
| 31 | 4 | healthy | CD4+CD25- 24 h CD3/IL2 stimulated |
| 32 | 4 | CLL | CD4+CD25- 24 h CD3/IL2 stimulated |
| 33 | 2 | healthy | CD4+CD25- untreated |
| 34 | 3 | healthy | CD4+CD25lowCD127+ untreated |
| 35 | 3 | healthy | CD4+CD25-CD127+ untreated |
| 36 | 4 | healthy | CD4+CD25-/lowCD127+ untreated |
| 37 | 4 | CLL | CD4+CD25+ untreated |
| 38 | 4 | healthy | CD4+CD25+ untreated |
| 39 | 4 | healthy | CD4+CD25+ 24 h CD3/IL2 stimulated |
| 40 | 4 | CLL | CD4+CD25+ 24 h CD3/IL2 stimulated |
| 41 | 4 | healthy | CD4+CD25+ untreated |
| 42 | 2 | healthy | CD4+CD25+ untreated |
| 43 | 2 | healthy | CD4+CD25+ 6 h cultured |
| 44 | 4 | healthy | CD4+CD25+ expanded |
| 45 | 4 | healthy | CD4+CD25+ expanded with Rapamycin |
| 46 | 4 | healthy | CD4+CD25+ expanded and 6 h shortterm CD3CD28 activation |

**Table 2: Primer murine qPCR**

| name | sequence | SEQ ID NO |
|---|---|---|
| B-actin Forward | CTAAGGCCAACCGTGAAAAG | 3 |
| B-actin Reverse | ACCAGAGGCATACAGGGACA | 4 |
| Foxp3 Forward | ACCACACTTCATGCATCAGC | 5 |
| Foxp3 Reverse | CCAGTGGCAGCAGAAGGT | 6 |
| SATB1 Forward | AGGAGTGCCCCCTTTCAC | 7 |
| SATB1 Reverse | TGCTGCTGAGACATTTGCAT | 8 |
| IFNgamma Fw. | CAGGAAGCGGAAAAGGAGT | 9 |
| IFNgamma Rev. | AAAATTCAAATAGTGCTGGCAGA | 10 |
| IL6 Forward | GCTACCAAACTGGATATAATCAGGA | 11 |
| IL6 Reverse | CCAGGTAGCTATGGTACTCCAGAA | 12 |

**Table 3: Primer ChIP-PCR**

| name | sequence | SEQ ID NO: |
|---|---|---|
| IL-7R promoter Forward | CAGGGAATATCCAGGAGGAA | 13 |
| IL-7R promoter Reverse | TGTGTGAGCCAGTGTGTATGAA | 14 |
| IL-7R intron 4 Forward | GAGGTGGCAGAAGAGTGGAG | 15 |
| IL-7R intron 4 Reverse | TGCATCACACTGCAAACAAA | 16 |
| SATB1 Forward | GCAGTAGAAAGGTGGGTTCTTC | 17 |
| SATB1 Reverse | TGGTGACGAAAGAGAAATAAATG | 18 |
| SATB1 Forward | GAAAGGTGGGTTCTTCTGAAGATA | 19 |
| SATB1 Reverse | GCAATGAATGCAGAATTACCTTT | 20 |
| EMSA Oligos | | |
| SATB1 binding site Fw. | GTATACAGTATGCAAACATAACTCACCATT | 21 |
| SATB1 binding site Rev. | AATGGTGAGTTATGTTTGCATACTGTATAC | 22 |
| SATB1 binding site (mut.) Fw. | GTATACAGTATCGTCGAGCAACTCACCATT | 23 |
| SATB1 binding site (mut.) Rev. | AATGGTGAGTTGCTCGACGATACTGTATAC | 24 |
| SATB1 binding competitor Fw. | TCAAAAATATTGAAGTGTTATCACATACAC | 25 |
| SATB1 binding competitor Rev. | GTGTATGTGATAACACTTCAATATTTTTGA | 26 |

**Tab. 4: Primer human qPCR**

| name | sequence | SEQ ID NO |
|---|---|---|
| SATB1 Forward | CGATGAACTGAAACGAGCAG | 27 |
| SATB1 Reverse | CGGAGGATTTCTGAAAGCAA | 28 |
| Foxp3 Forward | ACCTACGCCACGCTCATC | 29 |
| Foxp3 Reverse | TCATTGAGTGTCCGCTGCT | 30 |
| IL5 Forward | GGTTTGTTGCAGCCAAAGAT | 31 |
| IL5 Reverse | TCTTGGCCCTCATTCTCACT | 32 |
| IFNgamma Forward | CACTGAAGAAATCTTTCAGGGAAT | 33 |
| IFNgamma Reverse | CCGTCTTTCTTCTCCACACTTT | 34 |

Example 1: To identify regulatory circuits involved in FOXP3-mediated inhibition of T_{effector} cell differentiation a large transcriptome experiment was initiated comprising 171 individual samples in 48 experimental conditions of human resting or activated conventional FOXP3⁻ CD25⁻ T cells (T_{conv}) and natural regulatory CD25⁺ FOXP3⁺ T cells (nT_{reg}) (Fig. 6 and Table 1). Since miRNA represent an additional level of gene regulation we performed microRNA (miRNA) profiling of 753 human miRNAs in T_{reg} versus T_{conv} allowing us to calculate inverse correlations between gene expression and miRNA expression (total of 35 x 10⁶ correlations). Genes were filtered 1) by their differential expression between T_{reg} and T_{conv} samples, 2) by a significant inverse correlation between gene expression and those microRNAs significantly enriched in T_{reg}, and 3) by their gene ontology associated with e.g. transcriptional regulation, DNA methylation or histone modification. Of the 47 genes differentially expressed between T_{reg} and T_{conv}, the special AT-rich sequence-binding protein 1 (SATB1) (Fig.1a) was among the genes with the most significant inverse correlation to a particular miRNA, namely miR-155 (Fig 1b), a miRNA which was significantly enriched in T_{reg} (Fig. 7). In murine TH2 clones, SATB1 has been shown to function as a global transcriptional regulator specifically anchoring the looped topology of the TH2 cytokine locus, a pre-requisite for the induction of certain TH2 cytokines (Cai, S. et al., Nat. Genet 38-1278-1288 (2006); Pipkin, M.E., Monticell, S., Immunology 124:23-32 (2008)). Since SATB1-deficient thymocytes do not develop beyond the double-positive stage (Alvarez, J.D. et al., Genes dev. (14: 521-535 (2000); Cai, S. et al., Nat. Genet. 34:42-51 (2003)) the role of SATB1 in peripheral T cells, particularly in T_{reg}, is still elusive.

Reduced SATB1 mRNA and protein expression in nT_{reg} was confirmed for a larger set of samples by qRT-PCR (Fig. 1c),Western blotting (Fig 1d) and intracellular flow cytometry using a directly conjugated SATB1 mAb (Fig 1e). In nT_{reg} SATB1 can be regulated by exogenous signals such as T cell receptor (TCR) and costimulation (here CD28), however expression never exceeded levels observed in resting T_{conv} (Fig 1f). TGFß significantly decreases SATB1 expression both in nT_{reg} and in T_{conv}, while only stimulated T_{conv} but not nT_{reg} expressed TH1 and TH2 cytokines (Fig. 1f and Fig. 7). Since TGFß is the major stimulus for the induction of adaptive or induced T_{reg} cells (iT_{reg}) (Chen, W. et al., J. Exp. Med. 198:1875-1886 (2003)), we assessed SATB1 regulation under these conditions. Naive human CD25⁻ CD45RA⁺ T cells were stimulated via TCR and CD28 with or without TGFß. T cells stimulated in the presence of TGFß exhibited the hallmarks of iT_{reg}, namely significant expression of FOPX3 mRNA, and protein as well as T cell suppressive function (Fig. 8). As previously reported by others, TCR and CD28 stimulation (Tₛₜᵢₘ) could also induce transient FOXP3 and suppressive function, however, this was variable and always inferior to iT_{reg}. In contrast, when assessing SATB1 expression, significantly enhanced expression was only observed in Tₛₜᵢₘ but not in iT_{reg} (Fig. 2a,b). SATB1 mediated chromatin remodelling via modification of histone acetylation and nucleosome placement has been linked to reduced IL-2RA gene transcription (Yasui, D. et al., Nature 419:641-645 (2002)). In line with these previous findings, we observe the highest upregulation of CD25 in iT_{reg} in the absence of SATB1 induction (Fig. 2c). In contrast, TH1 and TH2 cytokines are only produced in cells with significantly increased SATB1 expression (Fig. 2d). Taken together, reduced expression of SATB1 seems to be a novel hallmark of both iT_{reg} and nT_{reg} in humans.

SATB1 expression was also significantly reduced in flow-sorted murine T_{reg} derived from DEREG mice (Lahl, K. et al., J. Exp. Med. 204:57-63 (2007)) (Fig. 3a, b and Fig. 9) suggesting conserved regulation of SATB1 in T_{reg}. These findings were further supported by four-color immunohistochemistry of thymic tissue showing reduced SATB1 expression in T_{reg} *in vivo* (Fig. 3c). Even more striking, male DEREG mice harbouring a mutated FOXP3 (DEREG x scurfy), displayed a significantly increased SATB1 expression in T_{reg} (Fig. 3c,d). These findings prompted us to re-analyze a previous transcriptome analysis (Williams, L.M. et al., Nat. Immunol 8:277-284 (2007)) in mice transgenic for a mutated FOXP3 gene in T_{reg}, which revealed a similar relation between loss-of-function of FOXP3 and increase of SATB1 expression in murine T_{reg} (Fig. 10). In female DEREG mice heterozygous for the mutated scurfy allele we were able to demonstrate increased SATB1 expression in FOXP3-deficient T_{reg} in comparison to T_{reg} with intact FOXP3 further supporting that SATB1 expression is FOXP3 dependent in T_{reg} *in vivo* (Fig. 3e, f).

After establishing reduced SATB1 expression in human and murine T_{reg} *in vivo* and *in vitro*, we next attempted to uncover the molecular mechanisms responsible for reduced SATB1 expression in T_{reg}. First we assessed the potential of FOXP3 directly repressing SATB1. A search for conserved binding sites of FOXP3 within the genomic locus of the murine and human SATB1 revealed a FOXP3 motif within the second intron (Fig. 4a and Fig. 11). Re-analysis of previously reported ChIP-Chip analysis of murine T_{reg} (Zheng, Y. et al., Nature 445:936-940 (2007)) suggested significant binding of FOXP3 to this conserved region within the murine SATB1 locus. Using EMSA (electrophoretic mobility-shift assays, Fig. 4b) and a FOXP3-specific ChIP (chromatin immunoprecipitation) (Fig. 4c), we were able to demonstrate FOXP3 binding to the SATB1 locus *in vitro* and *in vivo* in highly purified human nT_{reg}. To probe the functional consequences of FOXP3 binding to the SATB1 locus, we cloned a reporter construct composed of the 180 bp of the second SATB1 intron adjacent to the FOXP3-binding site fused to a luciferase reporter gene. Expression of this construct in HEK293T cells resulted in constitutive luciferase activity and co-transfection of human FOXP3 led to a significant decrease in activity (Fig. 4d). This decreased luciferase activity was not observed following mutation of the FOXP3 binding site.

To assess the consequence of FOXP3 depletion on SATB1 expression in human nT_{reg} loss-of-function experiments silencing FOXP3 by siRNA were performed. This resulted in a significant loss of FOXP3 expression and suppressive function of nT_{reg} (Fig. 12). A significant increase of SATB1 expression was evident in FOXP3 depleted human nT_{reg} cells (Fig 4e), which was accompanied by an induction of TH1 (IFN-γ) and TH2 (IL-4 and IL-5) cytokines (Fig 4f, g). In line with this finding an increase of TH1 and TH2 cytokines *in vivo* was observed in T_{reg} from DEREG x scurfy mice (Fig. 13). Additional knockdown of SATB1 in human T_{reg} with a silenced FOXP3 gene resulted in decreased induction of T-helper cytokines (Fig. 4h) indicating that the release of T_{effector} cytokines in FOXP3-deficient T_{reg} is dependent on SATB1.

When performing gain-of-function experiments overexpressing FOXP3 in T_{conv} cells, a reduced expression of SATB1 was observed further supporting the regulatory effect of FOXP3 on SATB1 expression (Fig. 4i) which was accompanied by a concomitant decrease in cytokine mRNA expression (Fig. 14). Together, these findings establish that reduced SATB1 expression is not only a hallmark of T_{reg} but a consequence of direct inhibition by FOXP3. FOXP3-mediated suppression of SATB1 is required to prevent the expression of T_{effector} cytokines in murine and human T_{reg}.

The potential of miR-155 to control SATB1 expression was assessed. MiR-155 has been linked to normal B- and T-cell development and differentiation but also tumorigenesis (Rodriguez, A. et al., Science 316:608-611 (2007; Thai, T.H. et al., Science 316:604-608; Eis, P.S. et al., Proc. Natl. Acad. Scie. USA 102:3627-3632 (2005)). More recently it was suggested as a downstream target of FOXP3 (Zheng, Y. et al., Nature 445:936-940 (2007); Lu, L.F. et al., Immunity 30:80-91 (2009)). MiR-155 is highly expressed in human T cells, particularly in nT_{reg} (Fig. 5a) but also in iT_{reg} (Fig. 15) (Cobb, B.S. et al., J. Exp. Med. 203:2519-2527 (2006)). SiRNA-mediated knockdown of FOXP3 in human T_{reg} cells resulted in a marked decrease in miR-155 expression while FOXP3 overexpression induced miR-155 expression corroborating the regulation of miR-155 by FOXP3 (Fig. 16). Binding of seed-matched sites was computationally predicted using miRBase Targets (Griffiths-Jones, S. et al., Nucleic Acids Res. 36:D154-158 (2008)), miRanda (Betel, D. et al., Nucleic Acids Res. 36:D149-153 (2008)), PicTar (Krek, A. et al., Nat. Genet. 37:495-500 (2005)), and TargetScan (Lewis, B.P. et al., Cell 115:787-798 (2003)) (Fig. 5b). We fused the SATB1 3' UTR to a luciferase reporter gene and determined luciferase activity in 293T cells transfected with synthetic miR-155. Overexpression of miR-155 significantly repressed luciferase activity, whereas a control miRNA, lacking a predicted binding motif had no effect (Fig. 5c). In contrast, mutation of the miR-155 binding motif resulted in a restoration of luciferase activity (Fig. 5c). Loss-of-function experiments by antisense oligonucleotide mediated inhibition of miR-155 in primary human T_{reg} cells lead to a significant increase in SATB1 mRNA expression (Fig. 5d). Altogether, SATB1 expression is not only reduced on a transcriptional level by direct binding of FOXP3 to the genomic locus of SATB1 but reduced expression is further stabilized by the FOXP3 regulated miR-155.

To study epigenetic regulation of the SATB1 locus a specific region for methylation analysis based on CpG density which aligns to the predicted SATB1 promoter 1600 base pairs upstream of exon 1 was selected. As a control, the well-described site of differential methylation at the FOXP3 locus (Floess, S. et al., PloS Biol. 5:e38 (2007); Baron, U. et al., eur. J. Immunol. 37:2378-2389 (2007)) was also analyzed by bisulphite sequencing (Fig. 17). While there was a clear difference in methylation of the FOXP3 locus between T_{reg} and T_{conv}, the SATB1 locus was similarly demethylated in both cell types (Fig. 5e). This finding supports that methylation of CpG motifs within a selected element of the SATB1 locus does not contribute to the impeded expression of SATB1 in T_{reg} cells. By reanalyzing a ChIP-sequencing dataset for histone methylation in T-cell subsets (Wei, G. et al., Immunity 30:155-167 (2009)), H3 trimethylation at lysine residue 4 (H3K4me3), which is permissive for gene transcription, was detectable in iT_{reg} and nT_{reg} and further elevated in naïve T cells and T_{effector.} H3 trimethylation at lysine residue 27 (H3K27me3) which has been associated with gene silencing was absent in all T-cell subset (Fig. 18a, b). Taken together, the lack of silencing histone and DNA methylation is compatible with accessibility of the SATB1 locus for gene transcription in T_{reg}.

In conclusion, it was established FOXP3-mediated transcriptional and miR-155-mediated posttranscriptional repression of the global chromatin organizer SATB1 in iT_{reg} and nT_{reg} in man and mice (Figs. 19 and 20). These data implicate that T_{reg} compose a network of continuously activated regulatory circuits suppressing major target genes such as SATB1 required for the differentiation of T_{effector}. An active and continuous blockade of T_{effector} function instead of terminal T_{reg} differentiation allows T cells a higher degree of plasticity. This might be particularly interesting in situations where there is a temporary induction of adaptive T_{reg} cells that can gain T_{effector} function once FOXP3 is switched off again. Recent data concerning regulation of transcription factors such as IRF4 (Zheng, Y. et al., Nature (2009)) or epigenetic regulation of T lineage-associated transcription factors (Wei, G. et al., Immunity 30:155-167 (2009)) are also in line with a model of continuously active regulatory networks shaping the overall function of T cells in the periphery as an alternative to terminal differentiation.

Example 2: To identify regulatory circuits involved in FOXP3-mediated inhibition of T_{effector} differentiation, whole transcriptome analysis of human resting or activated conventional FOXP3⁻CD25⁻ T cells (T_{conv}) and natural regulatory CD25⁺FOXP3⁺ T cells (nT_{reg}) was performed (Fig. 25 and Table 1). Of the 47 genes specifically differentiating between T_{reg} and T_{conv}, special AT-rich sequence-binding protein 1 (SATB1) (Fig. 21a) was among the genes that were always expressed at significantly lower levels in T_{reg} compared to T_{conv}. Re-assessment of transcriptome data from previous reports confirmed our observation of SATB1 to be a potential target of FOXP3-mediated repression (Pfoertner, S. et al., Genome Biol 7, R54 (2006); Zheng, Y. et al., Nature 445, 936-940 (2007); Sugimoto, N. et al., Int Immunol 18, 1197-1209 (2006)). SATB1 is a transcription factor and chromatin organizer essential for controlling a large number of genes participating in T-cell development and activation (Alvarez, J. D. et al., Genes Dev 14, 521-535 (2000)). SATB1 regulates gene expression by directly recruiting chromatin modifying factors (Yasui, D. et al., Nature 419, 641-645 (2002)) and anchoring matrix attachment regions to the nuclear matrix (Cai, S. et al., Nat Genet 34, 42-51 (2003)). In murine TH2 clones, SATB1 has been shown to function as a global transcriptional regulator specifically anchoring the looped topology of the TH2 cytokine locus, a pre-requisite for the induction of certain TH2 cytokines (Cai, S. et al., Nat Genet 38, 1278-1288 (2006)). Since SATB1-deficient thymocytes do not develop beyond the double-positive stage (Alvarez, J. D. et al. Genes Dev 14, 521-535 (2000); Cai, S. et al., Nat Genet 34, 42-51 (2003)) the role of SATB1 in peripheral T cells, including T_{reg}, is still elusive. Next we validated the initial transcriptome data in a larger set of samples by qRT-PCR (Fig. 21b), western blotting (Fig. 21c), and intranuclear staining (Fig. 21d) and could clearly demonstrate reduced SATB1 mRNA and protein expression in human nT_{reg}. As increase in SATB1 expression was previously linked to CD4⁺ T-cell activation/differentiation (Lund, R. et al., Eur J Immunol 35, 3307-3319 (2005)) we assessed SATB1 regulation in T_{conv} and T_{reg} during activation via the T-cell receptor (using CD3 mAbs) in presence of costimulation (CD28 mAbs) or the cytokine interleukin-2. Flow-cytometric analysis of SATB1 expression established a stimulation dependent upregulation of SATB1 in T_{conv} while resting T_{reg} showed significantly lower SATB1 expression and lacked stimulation dependent upregulation (Fig. 21e and Fig. 26).

Since TGFβ is a major stimulus for the induction of adaptive or induced T_{reg} (iT_{reg}) (Chen, W. et al., J Exp Med 198, 1875-1886 (2003)), we assessed SATB1 regulation under these conditions. Naïve human CD25⁻CD45RA⁺ T cells were stimulated via TCR and CD28 with or without TGFβ. T cells stimulated in the presence of TGFβ exhibited the hallmarks of iT_{reg}, namely significant expression of FOPX3 mRNA, and protein as well as T-cell suppressive function (data not shown). As previously reported, TCR and CD28 stimulation (Tₛₜᵢₘ) could also induce transient FOXP3 expression and suppressive function, however, this was variable and always inferior to iT_{reg}. In contrast, when assessing SATB1 expression, significantly enhanced expression was only observed in Tₛₜᵢₘ but not in iT_{reg} or naïve T cells after 5 days of culture (Fig. 21f,g). In line with this observation, TH1 and TH2 cytokines are only produced in cells with significantly increased SATB1 expression (Fig. 21h). Taken together, reduced expression of SATB1 seems to be a novel hallmark of both iT_{reg} and nT_{reg} in humans.

Next, SATB1 expression in murine thymic T_{reg} in mice expressing GFP under the FOXP3 promoter was assessed (Lahl, K. et al., J Exp Med 204, 57-63 (2007)) using qPCR, western blotting, flow cytometry, and confocal microscopy. In vivo, SATB1 mRNA and protein expression was always lower in T_{reg} (Fig. 21i-k and Fig. 27) suggesting conserved regulation of SATB1 in human and murine T_{reg}. Similar to previous reports we observed nuclear localization of SATB1 in FOXP3⁻ thymocytes forming a cage-like structure within the nucleus (Cai, S. et al., Nat Genet 34, 42-51 (2003)). In FOXP3⁺ GFP⁺ T_{reg} localization and distribution of SATB1 was comparable, however the fluorescence intensity was always lower (Fig. 21j and Fig. 27c, d).

To further elucidate SATB1 as a potential FOXP3-target gene, we analyzed T_{reg} from male DEREG mice harbouring a spontaneously mutated FOXP3 (DEREG x scurfy) allele. Flow-sorted T_{reg} from these animals displayed a significantly increased SATB1 expression in T_{reg} compared to FOXP3-competent T_{reg} (Fig. 21i). These findings were validated by re-assessment of three transcriptome data sets (GSE18387, GSE6681, GSE11775) (Williams, L. M. & Rudensky, A. Y., Nat Immunol 8, 277-284 (2007); Anz, D. et al., J Immunol 184, 939-946; Kuczma, M. et al., J Immunol 183, 3731-3741 (2009)) derived from mice with a mutated FOXP3 gene in T_{reg} (data not shown). Overall, loss of function of FOXP3 was associated with increased expression of SATB1 in these murine model systems. To further address the role of FOXP3 control on SATB1 expression in T_{reg} in vivo, we assessed SATB1 expression in so-called 'exFOXP3 T_{reg}' introduced by Bluestone and colleagues (Zhou, X. et al., Nat Immunol 10, 1000-1007 (2009)). In this murine model cells can be identified that have lost FOXP3 expression during their life span and regained effector function. When assessing these 'exFOXP3 T_{reg}' under resting conditions, SATB1 expression was still significantly lower than in Tconv (data not shown).

Cell-autonomous control of SATB1 by FOXP3 was further supported by findings in female DEREG mice heterozygous for the mutated scurfy allele. These mice harbour both T_{reg} with normal FOXP3 function and T_{reg} with mutated FOXP3. Assessment on the single cell level using flow cytometry and confocal microscopy again revealed increased SATB1 expression in T_{reg} with mutated FOXP3 but not in T_{reg} harbouring normal FOXP3 (Fig. 22j, k).

Inverse correlation between FOXP3 and SATB1 expression in murine and and human T_{reg} cells strongly suggested that FOXP3 might act directly as a transcriptional repressor of the SATB1 locus. We performed FOXP3-ChIP tiling arrays of human natural T_{reg} (Fig. 22a) as well as bioinformatic in silico prediction to identify 8 sides for qPCR validation which were located -5kb upstream of the TSS as well as in the genomic locus of SATB1 (Fig. 22b). FOXP3 binding within the promoter region or genomic locus of SATB1 in T_{reg} was demonstrated by ChIP-coupled quantitative PCR (ChIP-qPCR) (Fig. 22c) and electrophoretic mobility-shift assays (data not shown). Binding affinity of FOXP3 was assessed for the six most 3' FOXP3 binding elements by in vitro DNA-protein interaction studies revealing K_{D} between x-y µM (Fig. 22d). Nonspecific binding was ruled out by using mutated FOXP3 binding motives, which showed significantly higher K_{D} for FOXP3 binding further supporting a specific binding of FOXP3 to numerous sites at the genomic SATB1 locus.

To probe the functional consequences of FOXP3 binding to the SATB1 locus, a luciferase reporter reporter assays was performed for six of the FOXP3 binding regions. FOXP3 binding regions were cloned between a minP promoter element and a luciferase reporter gene. Expression of these constructs resulted in luciferase activity and co-transfection of human FOXP3 led to a significant decrease in activity for five of the six regions analyzed (Fig. 22e). Using the mutated FOXP3 binding motives within these regions decreased luciferase activity was rescued (Fig. 22e) indicating that SATB1 expression is actively repressed by binding of FOXP3 to several functional binding sites within the genomic SATB1 locus.

To assess the consequence of FOXP3 depletion on SATB1 expression in human nT_{reg}, we first performed loss-of-function experiments silencing FOXP3 by siRNA. This resulted in a significant loss of FOXP3 expression and suppressive function of nT_{reg} (Fig. 28). A small but significant increase of SATB1 expression was already evident in unstimulated FOXP3-depleted human nT_{reg} (Fig. 2f), but this was significantly enhanced when T_{reg} where stimulated via TCR with costimulation or IL-2. This increase in SATB1 expression was accompanied by an induction of TH1 (IFN-γ) and TH2 (IL-4 and IL-5) cytokines (Fig. 22g, h). In line with this finding an increase of TH1 and TH2 cytokines in vivo was observed in T_{reg} from DEREG x scurfy mice (Fig. 29). Additional knockdown of SATB1 in human T_{reg} with a silenced FOXP3 gene resulted in a significantly decreased induction of T-helper cytokines (Fig. 22i) indicating that the release of T_{effector} cytokines in FOXP3-deficient T_{reg} is dependent on SATB1. Gain-of-function experiments overexpressing FOXP3 in T_{conv} resulted in a reduced expression of SATB1 (Fig. 22j), which was accompanied by a concomitant decrease in cytokine mRNA expression (Fig. 30). Together, these findings establish that reduced SATB1 expression is not only a hallmark of T_{reg} but a consequence of direct inhibition by FOXP3. FOXP3-mediated suppression of SATB1 is required to prevent the expression of T_{effector} cytokines in murine and human T_{reg}.

Blockade of T_{effector} cytokines is necessary but not sufficient for T_{reg} to exert suppressive function. To determine whether suppression of SATB1 is necessary for T_{reg} suppressive function we overexpressed SATB1 in human natural CD25^{high} FOXP3⁺ T_{reg} and assessed suppressive function in comparison to control-vector transduced T_{reg}. In sharp contrast to control-transduced T_{reg}, SATB1-expressing T_{reg} cells lost suppressive function (Fig. 23a). At the same time, these cells gained expression of TH1 (IFN-γ) and TH2 (IL-4) cytokines (Fig. 23b) suggesting a reprogramming of T_{reg} cells into T_{effector} once regulation of SATB1 is lost in T_{reg}.

To estimate the overall changes in SATB1 overexpressing T_{reg} cells whole transcriptome analysis was performed. A total of 100 genes were significantly increased in SATB1^{high} T_{reg}. whereas 21 were decreased (Fig. 23c). Cross annotation analysis of the differentially expressed genes revealed that 29% of the changed genes were primarily linked with T-cell activation, 20% were associated with expression in T_{conv} (in comparison to T_{reg}), 16% were classified as activation genes of T_{conv}. The remaining genes (35%) showed no particular association with T-cell function or lineage and were classified as SATB1-specific (Fig. 23d). Further, assessment transcriptional changes for enrichment of genes associated with TH1, TH2, and TH17 differentiation revealed induction of many genes involved in T_{effector} differentiation in SATB1^{high} T_{reg} (Fig. 23e).

Another level of SATB1 regulation might be achieved by epigenetic control of the SATB1 locus, e.g. by DNA methylation at CpG-rich sites. CpG density analysis of the SATB1 locus revealed three CpG rich-sites upstream of exon 1 (Fig. 24a) which were analyzed by bisulphite sequencing. The site of differential methylation at the FOXP3 locus (Floess, S. et al., PLoS Biol 5, e38 (2007)) was used as positive control (Fig. 31). While there was a clear difference in methylation of the FOXP3 locus between T_{reg} and T_{conv}, the SATB1 locus was similarly demethylated in both cell types (Fig. 24a). Similarly, when analyzing a genome-wide ChIP-sequencing dataset for histone methylation in T-cell subsets (Zheng, Y. et al., Nature 445, 936-940 (2007)), H3 trimethylation at lysine residue 4 (H3K4me3), permissive for gene transcription, was detectable in iT_{reg} and nT_{reg} at the SATB1 locus and further elevated in T_{effector} (Fig. 32). H3 trimethylation at lysine residue 27 (H3K27me3), which is associated with gene silencing, was low or absent in all T-cell subsets (Fig. 32). The lack of silencing histone and DNA methylation is compatible with accessibility of the SATB1 locus for gene transcription in T_{reg}.

In addition to direct FOXP3-mediated suppression, microRNAs (miRNAs) might represent an additional post-transcriptional level of gene regulation modulating SATB1 expression in human Treg. Performing miRNA profiling of 753 human miRNAs in T_{reg} versus T_{conv} allowed to establish differentially expressed miRNAs in T_{reg} and to calculate inverse correlations between SATB1 gene expression and miRNA expression (Fig. 24a). Using this approach as well as computational prediction of miRNA binding of seed-matched sites using miRBase Targets, miRanda, PicTar, and TargetScan (Fig. 24b), 5 miRNAs were identified that were differentially expressed between T_{reg} and T_{conv} (Fig. 24c) showing a significant inverse correlation between SATB1 and miRNA expression (Fig. 24d). Of these 5 miRNAs, miR-155, miR-21, and miR-7 are direct targets of FOXP3 as previously reported for miR-155 (Zheng, Y. et al., Nature 445, 936-940 (2007); Lu, L. F. et al., Immunity 30, 80-91 (2009)) and miR-21 and confirmed by FOXP3-ChIP tiling arrays (Fig. 24e) as well as functional analysis (Simon Barry, unpublished data).

For the assessment of functionally relevant binding of the miRNAs to the 3' UTR of the SATB1 mRNA we fused the SATB1 3' UTR to a luciferase reporter gene and determined luciferase activity in cells transfected with synthetic miRNAs. Expression of any of the 5 miRNAs significantly repressed constitutive luciferase activity, whereas a control miRNA, lacking a binding motif on the 3' UTR of SATB1, had no effect (Fig. 24f). Mutation of the respective binding motifs resulted in restoration of luciferase activity (Fig. 24f). Exemplary, we next assessed the potential of a single miRNA, miR-155, to control SATB1 expression. Loss-of-function experiments by antisense oligonucleotide-mediated inhibition of miR-155 in primary human T_{reg} resulted only in minor differences in SATB1 mRNA expression (Fig. 33) clearly indicating that the loss of a single miRNA cannot rescue SATB1 expression. Complete loss of all miRNAs, however, as achieved in mice by a T_{reg}-specific deletion of DICER (Zhou (2008)) clearly leads to upregulation of SATB1 on both mRNA and protein level using 2 independent mouse models (Fig 24g and Fig. 34). So far our data show that SATB1 expression is reduced in T_{reg} both by direct binding of FOXP3 to the genomic locus of SATB1 and binding of FOXP3-regulated miRNAs to the 3' UTR of the SATB1 mRNA.

In conclusion, FOXP3-mediated transcriptional and miRNA-mediated posttranscriptional repression of the global chromatin organizer SATB1 in iT_{reg} and nT_{reg} in humans and mice was establish. Repression of SATB1 is required for sustaining suppressive function of T_{reg} and inhibition of effector function in these cells (Fig. 36). Further, these data implicate that T_{reg} compose a network of continuously activated regulatory circuits suppressing major target genes such as SATB1 required for the differentiation of T_{effector}. An active and continuous blockade of T_{effector} function instead of terminal T_{reg} differentiation allows T cells a higher degree of plasticity. This might be particularly interesting in situations where there is a temporary induction of adaptive T_{reg} cells that can gain T_{effector} function once FOXP3 is switched off again (Zhou (2009)). Recent data concerning regulation of transcription factors such as IRF4 (Zheng, Y. et al., Nature (2009)) or epigenetic regulation of T lineage-associated transcription factors (Wei, G. et al., Immunity 30, 155-167 (2009)) are also in line with a model of continuously active regulatory networks shaping the overall function of T cells in the periphery as an alternative to terminal differentiation.

### Sequence Listing - Free Text

| SEQ ID | Designation |
|---|---|
| 1/2 | >gi\| 33356175\| ref\| NM_002971.2\| Homo sapiens SATB homeobox 1 (SATB1), mRNA and CDS |
| 3-34 | primer |
| 35-42 | FOXP3 binding sites in the SATB1 locus over several mammals |
| 43 | human miR-155 binding site |

## Claims

1. A method of detecting unstable regulatory T cells in a population of regulatory T cells that have the potential for converting to effector T cell functionality, which method comprises detecting cells with elevated levels of SATB1 protein expression in the population of T cells.

2. The method of claim 1, wherein the cells with elevated levels of SATB1 protein expression in the population of T cells are detected by a method comprising
(a) contacting the cell population with one or more ligands that specifically bind to SATB1, and
(b) identifying the regulatory T cells in the cell population due to a significant reduction of binding with the SATB1-binding ligands as compared to binding of said ligands with the other cells in the cell population.

3. The method of claim 1 or 2, which is suitable for quality control of regulatory T cell populations.

4. The method of claim 2, wherein the ligands are antibodies or fragments thereof, preferably the ligands are monoclonal antibodies or fragments thereof.

5. The method of claim 2 or 4, wherein the ligands/antibodies carry functional moieties including, but not limited to labels, dyes and toxins.

6. The method of claim 2, 4 or 5, wherein the cell population is selected from cell culture, whole blood and fractions of whole blood and/or the cell population comprises mammalian cells including human cells.

7. The method of claim 6, further comprising contacting the human cell population with one or more ligands that specifically bind to CD4, CD25 and/or CD127 on the T cells.

8. The method of claim 6 or 7, further comprising assaying for FOXP3 expression.

9. Use of the ligand, the antibody or antibody fragment of claim 2, 4 or 5 for identifying unstable regulatory T cells in a cell population.

## Patentansprüche

1. Verfahren zum Nachweisen instabiler regulatorischer T-Zellen in einer Population von regulatorischen T-Zellen, die das Potential zur Umwandlung zu Effektor-T-Zell-Funktionalität aufweisen, wobei das Verfahren das Nachweisen von Zellen mit erhöhten Niveaus von SATB1-Protein-Expression in der Population von T-Zellen umfasst.

2. Verfahren gemäß Anspruch 1, wobei die Zellen mit erhöhten Niveaus von SATB1-Protein-Expression in der Population von T-Zellen mit einem Verfahren nachgewiesen werden, das Folgendes umfasst:
(a) In-Kontakt-Bringen der Zellpopulation mit einem oder mehreren Liganden, die spezifisch an SATB1 binden; und
(b) Identifizieren der regulatorischen T-Zellen in der Zellpopulation aufgrund einer signifikanten Reduktion der Bindung an die SATB1-bindenden Liganden im Vergleich zur Bindung dieser Liganden an die anderen Zellen in der Zellpopulation.

3. Verfahren gemäß Anspruch 1 oder 2, das für die Qualitätskontrolle von Populationen regulatorischer T-Zellen geeignet ist.

4. Verfahren gemäß Anspruch 2, wobei die Liganden Antikörper oder Fragmente davon sind, wobei die Liganden vorzugsweise monoklonale Antikörper oder Fragmente davon sind.

5. Verfahren gemäß Anspruch 2 oder 4, wobei die Liganden/Antikörper funktionelle Struktureinheiten tragen, zu denen unter Anderen Marker, Farbstoffe und Toxine gehören.

6. Verfahren gemäß Anspruch 2, 4 oder 5, wobei die Zellpopulation aus einer Zellkultur, Vollblut und Fraktionen von Vollblut ausgewählt ist und/oder die Zellpopulation Säugerzellen einschließlich humaner Zellen umfasst.

7. Verfahren gemäß Anspruch 6, weiterhin umfassend das In-Kontakt-Bringen der humanen Zellpopulation mit einem oder mehreren Liganden, die spezifisch an CD4, CD25 und/oder CD127 auf den T-Zellen binden.

8. Verfahren gemäß Anspruch 6 oder 7, weiterhin umfassend das Testen auf FOXP3-Expression.

9. Verwendung des Liganden, Antikörpers oder Antikörperfragments gemäß Anspruch 2, 4 oder 5 zum Identifizieren instabiler regulatorischer T-Zellen in einer Zellpopulation.

## Revendications

1. Procédé de détection des lymphocytes T régulateurs instables dans une population de lymphocytes T régulateurs qui ont le potentiel de conversion pour une fonctionnalité de lymphocytes T effecteurs, lequel procédé comprend la détection de cellules ayant des niveaux élevés d'expression de la protéine SATB1 dans la population de lymphocytes T.

2. Procédé selon la revendication 1, dans lequel les cellules ayant des niveaux élevés d'expression de la protéine SATB1 dans la population de lymphocytes T sont détectées par un procédé comprenant:
(a) la mise en contact de la population cellulaire avec un ou plusieurs ligands qui se lient spécifiquement à SATB1, et
(b) l'identification des lymphocytes T régulateurs dans la population cellulaire du fait d'une réduction significative de la liaison avec les ligands de liaison de SATB1 par rapport à la liaison desdits ligands avec les autres cellules dans la population cellulaire.

3. Procédé selon la revendication 1 ou 2, qui est adapté au contrôle de qualité des populations de lymphocytes T régulateurs.

4. Procédé selon la revendication 2, dans lequel les ligands sont des anticorps ou des fragments de ceux-ci, de préférence, les ligands sont des anticorps monoclonaux ou des fragments de ceux-ci.

5. Procédé selon la revendication 2 ou 4, dans lequel les ligands/anticorps comportent des groupements fonctionnels, y compris mais sans limitation, des marqueurs, de colorants et des toxines.

6. Procédé selon la revendication 2, 4 ou 5, dans lequel la population cellulaire est choisie parmi une culture cellulaire, du sang total et des fractions de sang total et/ou la population cellulaire comprend des cellules de mammifères, y compris des cellules humaines.

7. Procédé selon la revendication 6, comprenant en outre la mise en contact de la population de cellules humaines avec un ou plusieurs ligands qui se lient spécifiquement aux CD4, CD25 et/ou CD127 sur les lymphocytes T.

8. Procédé selon la revendication 6 ou 7, comprenant en outre l'analyse de l'expression de FOXP3.

9. Utilisation du ligand, de l'anticorps ou du fragment d'anticorps de la revendication 2, 4 ou 5 pour l'identification des lymphocytes T régulateurs instables dans une population cellulaire.


### SEQUENCE LISTING

<110> Rheinische Friedrich-Wilhelms-Universitaet Bonn Becton, Dickinson and Company
<120> Identification of Regulatory T Cells via the Global Gene Regulator SATB1
<130> 120944ep
<150> EP 10715520.2
   <151> 2010-04-06
<150> US 61/165,970
   <151> 2009-04-02
<160> 43
<170> PatentIn version 3.3
<210> 1
   <211> 3782
   <212> DNA
   <213> Homo sapiens
<220>
   <221> CDS
   <222> (246) .. (2537)
<400> 1
   ttctgccctt cccccgctta gggggcgggg ggtaggggaa aggaaaataa tacaatttca     60
   ggggaagtcg ccttcaggtc tgctgctttt ttattttttt tttttaatta aaaaaaaaaa    120
   ggacatagaa aacatcagtc ttgaacttct cttcaagaac ccgggctgca aaggaaatct    180
   cctttgtttt tgttatttat gtgctgtcaa gttttgaagt ggtgatcttt agacagtgac    240
   tgagt atg gat cat ttg aac gag gca act cag ggg aaa gaa cat tca gaa    290
         Met Asp His Leu Asn Glu Ala Thr Gln Gly Lys Glu His Ser Glu
         1               5                   10                  15
   atg tct aac aat gtg agt gat ccg aag ggt cca cca gcc aag att gcc      338
   Met Ser Asn Asn Val Ser Asp Pro Lys Gly Pro Pro Ala Lys Ile Ala
                   20                  25                  30
   cgc ctg gag cag aac ggg agc ccg cta gga aga gga agg ctt ggg agt      386
   Arg Leu Glu Gln Asn Gly Ser Pro Leu Gly Arg Gly Arg Leu Gly Ser
               35                  40                  45
   aca ggt gca aaa atg cag gga gtg cct tta aaa cac tcg ggc cat ctg      434
   Thr Gly Ala Lys Met Gln Gly Val Pro Leu Lys His Ser Gly His Leu
           50                  55                  60
   atg aaa acc aac ctt agg aaa gga acc atg ctg cca gtt ttc tgt gtg      482
   Met Lys Thr Asn Leu Arg Lys Gly Thr Met Leu Pro Val Phe Cys Val
       65                  70                  75
   gtg gaa cat tat gaa aac gcc att gaa tat gat tgc aag gag gag cat      530
   Val Glu His Tyr Glu Asn Ala Ile Glu Tyr Asp Cys Lys Glu Glu His
   80                  85                  90                  95
   gca gaa ttt gtg ctg gtg aga aag gat atg ctt ttc aac cag ctg atc      578
   Ala Glu Phe Val Leu Val Arg Lys Asp Met Leu Phe Asn Gln Leu Ile
               100                 105                 110
   gaa atg gca ttg ctg tct cta ggt tat tca cat agc tct gct gcc cag      626
   Glu Met Ala Leu Leu Ser Leu Gly Tyr Ser His Ser Ser Ala Ala Gln
           115                 120                 125
   gcc aaa ggg cta atc cag gtt gga aag tgg aat cca gtt cca ctg tct      674
   Ala Lys Gly Leu Ile Gln Val Gly Lys Trp Asn Pro Val Pro Leu Ser
       130                 135                 140
   tac gtg aca gat gcc cct gat gct aca gta gca gat atg ctt caa gat      722
   Tyr Val Thr Asp Ala Pro Asp Ala Thr Val Ala Asp Met Leu Gln Asp
       145                 150                 155
   gtg tat cat gtg gtc aca ttg aaa att cag tta cac agt tgc ccc aaa      770
   Val Tyr His Val Val Thr Leu Lys Ile Gln Leu His Ser Cys Pro Lys
   160                 165                 170                 175
   cta gaa gac ttg cct ccc gaa caa tgg tcg cac acc aca gtg agg aat      818
   Leu Glu Asp Leu Pro Pro Glu Gln Trp Ser His Thr Thr Val Arg Asn
               180                 185                 190
   gct ctg aag gac tta ctg aaa gat atg aat cag agt tca ttg gcc aag      866
   Ala Leu Lys Asp Leu Leu Lys Asp Met Asn Gln Ser Ser Leu Ala Lys
           195                 200                 205
   gag tgc ccc ctt tca cag agt atg att tct tcc att gtg aac agt act      914
   Glu Cys Pro Leu Ser Gln Ser Met Ile Ser Ser Ile Val Asn Ser Thr
       210                 215                 220
   tac tat gca aat gtc tca gca gca aaa tgt caa gaa ttt gga agg tgg      962
   Tyr Tyr Ala Asn Val Ser Ala Ala Lys Cys Gln Glu Phe Gly Arg Trp
       225                 230                 235
   tac aaa cat ttc aag aag aca aaa gat atg atg gtt gaa atg gat agt     1010
   Tyr Lys His Phe Lys Lys Thr Lys Asp Met Met Val Glu Met Asp Ser
   240                 245                 250                 255
   ctt tct gag cta tcc cag caa ggc gcc aat cat gtc aat ttt ggc cag     1058
   Leu Ser Glu Leu Ser Gln Gln Gly Ala Asn His Val Asn Phe Gly Gln
               260                 265                 270
   caa cca gtt cca ggg aac aca gcc gag cag cct cca tcc cct gcg cag     1106
   Gln Pro Val Pro Gly Asn Thr Ala Glu Gln Pro Pro Ser Pro Ala Gln
           275                 280                 285
   ctc tcc cat ggc agc cag ccc tct gtc cgg aca cct ctt cca aac ctg     1154
   Leu Ser His Gly Ser Gln Pro Ser Val Arg Thr Pro Leu Pro Asn Leu
       290                 295                 300
   cac cct ggg ctc gta tca aca cct atc agt cct caa ttg gtc aac cag     1202
   His Pro Gly Leu Val Ser Thr Pro Ile Ser Pro Gln Leu Val Asn Gln
       305                 310                 315
   cag ctg gtg atg gct cag ctg ctg aac cag cag tat gca gtg aat aga     1250
   Gln Leu Val Met Ala Gln Leu Leu Asn Gln Gln Tyr Ala Val Asn Arg
   320                 325                 330                 335
   ctt tta gcc cag cag tcc tta aac caa caa tac ttg aac cac cct ccc     1298
   Leu Leu Ala Gln Gln Ser Leu Asn Gln Gln Tyr Leu Asn His Pro Pro
               340                 345                 350
   cct gtc agt aga tct atg aat aag cct ttg gag caa cag gtt tcg acc     1346
   Pro Val Ser Arg Ser Met Asn Lys Pro Leu Glu Gln Gln Val Ser Thr
           355                 360                 365
   aac aca gag gtg tct tcc gaa atc tac cag tgg gta cgc gat gaa ctg      1394
   Asn Thr Glu Val Ser Ser Glu Ile Tyr Gln Trp Val Arg Asp Glu Leu
           370                 375                 380
   aaa cga gca gga atc tcc cag gcg gta ttt gca cgt gtg gct ttt aac      1442
   Lys Arg Ala Gly Ile Ser Gln Ala Val Phe Ala Arg Val Ala Phe Asn
       385                 390                 395
   aga act cag ggc ttg ctt tca gaa atc ctc cga aag gaa gag gac ccc      1490
   Arg Thr Gln Gly Leu Leu Ser Glu Ile Leu Arg Lys Glu Glu Asp Pro
   400                 405                 410                 415
   aag act gca tcc cag tct ttg ctg gta aac ctt cgg gct atg cag aat      1538
   Lys Thr Ala Ser Gln Ser Leu Leu Val Asn Leu Arg Ala Met Gln Asn
                   420                 425                 430
   ttc ttg cag tta ccg gaa gct gaa aga gac cga ata tac cag gac gaa      1586
   Phe Leu Gln Leu Pro Glu Ala Glu Arg Asp Arg Ile Tyr Gln Asp Glu
               435                 440                 445
   agg gaa agg agc ttg aat gct gcc tcg gcc atg ggt cct gcc ccc ctc      1634
   Arg Glu Arg Ser Leu Asn Ala Ala Ser Ala Met Gly Pro Ala Pro Leu
           450                 455                 460
   atc agc aca cca ccc agc cgt cct ccc cag gtg aaa aca gct act att      1682
   Ile Ser Thr Pro Pro Ser Arg Pro Pro Gln Val Lys Thr Ala Thr Ile
       465                 470                 475
   gcc act gaa agg aat ggg aaa cca gag aac aat acc atg aac att aat      1730
   Ala Thr Glu Arg Asn Gly Lys Pro Glu Asn Asn Thr Met Asn Ile Asn
   480                 485                 490                 495
   gct tcc att tat gat gag att cag cag gaa atg aag cgt gct aaa gtg      1778
   Ala Ser Ile Tyr Asp Glu Ile Gln Gln Glu Met Lys Arg Ala Lys Val
                   500                 505                 510
   tct caa gca ctg ttt gca aag gtt gca gca acc aaa agc cag gga tgg      1826
   Ser Gln Ala Leu Phe Ala Lys Val Ala Ala Thr Lys Ser Gln Gly Trp
               515                 520                 525
   ttg tgc gag ctg tta cgc tgg aaa gaa gat cct tct cca gaa aac aga      1874
   Leu Cys Glu Leu Leu Arg Trp Lys Glu Asp Pro Ser Pro Glu Asn Arg
           530                 535                 540
   acc ctg tgg gag aac ctc tcc atg atc cga agg ttc ctc agt ctt cct      1922
   Thr Leu Trp Glu Asn Leu Ser Met Ile Arg Arg Phe Leu Ser Leu Pro
       545                 550                 555
   cag cca gaa cgt gat gcc att tat gaa cag gag agc aac gcg gtg cat      1970
   Gln Pro Glu Arg Asp Ala Ile Tyr Glu Gln Glu Ser Asn Ala Val His
   560                 565                 570                 575
   cac cat ggc gac agg ccg ccc cac att atc cat gtt cca gca gag cag      2018
   His His Gly Asp Arg Pro Pro His Ile Ile His Val Pro Ala Glu Gln
                   580                 585                 590
   att cag caa cag cag cag caa cag caa cag cag cag cag cag cag cag      2066
   Ile Gln Gln Gln Gln Gln Gln Gln Gln Gln Gln Gln Gln Gln Gln Gln
               595                 600                 605
   gca ccg ccg cct cca cag cca cag cag cag cca cag aca ggc cct cgg      2114
   Ala Pro Pro Pro Pro Gln Pro Gln Gln Gln Pro Gln Thr Gly Pro Arg
           610                 615                 620
   ctc ccc cca cgg caa ccc acg gtg gcc tct cca gca gag tca gat gag      2162
   Leu Pro Pro Arg Gln Pro Thr Val Ala Ser Pro Ala Glu Ser Asp Glu
       625                 630                 635
   gaa aac cga cag aag acc cgg cca cga aca aaa att tca gtg gaa gcc      2210
   Glu Asn Arg Gln Lys Thr Arg Pro Arg Thr Lys Ile Ser Val Glu Ala
   640                 645                 650                 655
   ttg gga atc ctc cag agt ttc ata caa gac gtg ggc ctg tac cct gac      2258
   Leu Gly Ile Leu Gln Ser Phe Ile Gln Asp Val Gly Leu Tyr Pro Asp
                   660                 665                 670
   gaa gag gcc atc cag act ctg tct gcc cag ctc gac ctt ccc aag tac      2306
   Glu Glu Ala Ile Gln Thr Leu Ser Ala Gln Leu Asp Leu Pro Lys Tyr
               675                 680                 685
   acc atc atc aag ttc ttt cag aac cag cgg tac tat ctc aag cac cac      2354
   Thr Ile Ile Lys Phe Phe Gln Asn Gln Arg Tyr Tyr Leu Lys His His
           690                 695                 700
   ggc aaa ctg aag gac aat tcc ggt tta gag gtc gat gtg gca gaa tat      2402
   Gly Lys Leu Lys Asp Asn Ser Gly Leu Glu Val Asp Val Ala Glu Tyr
       705                 710                 715
   aaa gaa gag gag ctg ctg aag gat ttg gaa gag agt gtc caa gat aaa      2450
   Lys Glu Glu Glu Leu Leu Lys Asp Leu Glu Glu Ser Val Gln Asp Lys
   720                 725                 730                 735
   aat act aac acc ctt ttt tca gtg aaa cta gaa gaa gag ctg tca gtg      2498
   Asn Thr Asn Thr Leu Phe Ser Val Lys Leu Glu Glu Glu Leu Ser Val
                   740                 745                 750
   gaa gga aac aca gac att aat act gat ttg aaa gac tga gataaaagta       2547
   Glu Gly Asn Thr Asp Ile Asn Thr Asp Leu Lys Asp
               755                 760
   tttgtttcgt tcaacagtgc cactggtatt tactaacaaa atgaaaagtc caccttgtct   2607
   tctctcagaa aacctttgtt gttcattgtt tggccaatga atcttcaaaa acttgcacaa   2667
   acagaaaagt tggaaaagga taatacagac tgcactaaat gttttcctct gttttacaaa   2727
   ctgcttggca gccccaggtg aagcatcaag gattgtttgg tattaaaatt tgtgttcacg   2787
   ggatgcacca aagtgtgtac cccgtaagca tgaaaccagt gttttttgtt ttttttttag    2847
   ttcttattcc ggagcctcaa acaagcatta taccttctgt gattatgatt tcctctccta   2907
   taattatttc tgtagcactc cacactgatc tttggaaact tgccccttat ttaaaaaaaa   2967
   aaaagaaaaa aaagagtttg ttactctatt gtatgttaca aaagaactat agactgtgga   3027
   atgcagttta aagatgacat atgccaacaa atgccttgta ttatatggca ctgccgtaat   3087
   tcaaatttgt ttttattttg gaaataaaag ttcactgtac ttttttttca ttctcattgt    3147
   tacatgattt tttaaaaaaa ggaaaagaaa atgtgaaaca caatttagtc ctcattattt   3207
   atttgtagat cctgcagcat catgttgtaa ttaatttttt ggaagtttcc gttaaatgta   3267
   atattgcttc tcttgttacc atactgattc ttttctattt ataaatgtat tttgatgggc   3327
   agtaaaacaa agtgtcttaa aagttttaaa tagagaaaat gtgctttaca cagttgccta   3387
   taaaaagtgc tctatgttat ccaagcaatt catactataa gcttcactct tattgttgta   3447
   tgcaattttt actatcatgc aaataagctt aggtaaataa aactaataga tcaccttaga   3507
   aaattatgca attaatgtga aaataattga tgtttgcaat gtgtcttcct ttggtttaca   3567
   atcaatttta aagctacatc tgtataaaat ttctgtataa aggtgtattt cttttttatg   3627
   agtttatggc tatgaaaaca gctattttgt tacagctggc tgtttttata agtgtatcac   3687
   aattttcttt atgcagaaat gttctgacta ggagtggtta ttgactgtaa ctacacaatt   3747
   aaaattgttt gtatcgtaaa aaaaaaaaaa aaaaa                               3782
<210> 2
   <211> 763
   <212> PRT
   <213> Homo sapiens
<400> 2
   Met Asp His Leu Asn Glu Ala Thr Gln Gly Lys Glu His Ser Glu Met
   1               5                   10                  15
   Ser Asn Asn Val Ser Asp Pro Lys Gly Pro Pro Ala Lys Ile Ala Arg
               20                  25                  30
   Leu Glu Gln Asn Gly Ser Pro Leu Gly Arg Gly Arg Leu Gly Ser Thr
           35                  40                  45
   Gly Ala Lys Met Gln Gly Val Pro Leu Lys His Ser Gly His Leu Met
       50                  55                  60
   Lys Thr Asn Leu Arg Lys Gly Thr Met Leu Pro Val Phe Cys Val Val
   65                  70                  75                  80
   Glu His Tyr Glu Asn Ala Ile Glu Tyr Asp Cys Lys Glu Glu His Ala
                   85                  90                  95
   Glu Phe Val Leu Val Arg Lys Asp Met Leu Phe Asn Gln Leu Ile Glu
               100                 105                 110
   Met Ala Leu Leu Ser Leu Gly Tyr Ser His Ser Ser Ala Ala Gln Ala
           115                 120                 125
   Lys Gly Leu Ile Gln Val Gly Lys Trp Asn Pro Val Pro Leu Ser Tyr
       130                 135                 140
   Val Thr Asp Ala Pro Asp Ala Thr Val Ala Asp Met Leu Gln Asp Val
   145                 150                 155                 160
   Tyr His Val Val Thr Leu Lys Ile Gln Leu His Ser Cys Pro Lys Leu
                   165                 170                 175
   Glu Asp Leu Pro Pro Glu Gln Trp Ser His Thr Thr Val Arg Asn Ala
   180 185 190
   Leu Lys Asp Leu Leu Lys Asp Met Asn Gln Ser Ser Leu Ala Lys Glu
   195 200 205
   Cys Pro Leu Ser Gln Ser Met Ile Ser Ser Ile Val Asn Ser Thr Tyr
   210 215 220
   Tyr Ala Asn Val Ser Ala Ala Lys Cys Gln Glu Phe Gly Arg Trp Tyr
   225 230 235 240
   Lys His Phe Lys Lys Thr Lys Asp Met Met Val Glu Met Asp Ser Leu
   245 250 255
   Ser Glu Leu Ser Gln Gln Gly Ala Asn His Val Asn Phe Gly Gln Gln
   260 265 270
   Pro Val Pro Gly Asn Thr Ala Glu Gln Pro Pro Ser Pro Ala Gln Leu
   275 280 285
   Ser His Gly Ser Gln Pro Ser Val Arg Thr Pro Leu Pro Asn Leu His
   290 295 300
   Pro Gly Leu Val Ser Thr Pro Ile Ser Pro Gln Leu Val Asn Gln Gln
   305 310 315 320
   Leu Val Met Ala Gln Leu Leu Asn Gln Gln Tyr Ala Val Asn Arg Leu
   325 330 335
   Leu Ala Gln Gln Ser Leu Asn Gln Gln Tyr Leu Asn His Pro Pro Pro
   340 345 350
   Val Ser Arg Ser Met Asn Lys Pro Leu Glu Gln Gln Val Ser Thr Asn
   355 360 365
   Thr Glu Val Ser Ser Glu Ile Tyr Gln Trp Val Arg Asp Glu Leu Lys
   370 375 380
   Arg Ala Gly Ile Ser Gln Ala Val Phe Ala Arg Val Ala Phe Asn Arg
   385 390 395 400
   Thr Gln Gly Leu Leu Ser Glu Ile Leu Arg Lys Glu Glu Asp Pro Lys
   405 410 415
   Thr Ala Ser Gln Ser Leu Leu Val Asn Leu Arg Ala Met Gln Asn Phe
   420 425 430
   Leu Gln Leu Pro Glu Ala Glu Arg Asp Arg Ile Tyr Gln Asp Glu Arg
   435 440 445
   Glu Arg Ser Leu Asn Ala Ala Ser Ala Met Gly Pro Ala Pro Leu Ile
   450 455 460
   Ser Thr Pro Pro Ser Arg Pro Pro Gln Val Lys Thr Ala Thr Ile Ala
   465 470 475 480
   Thr Glu Arg Asn Gly Lys Pro Glu Asn Asn Thr Met Asn Ile Asn Ala
   485 490 495
   Ser Ile Tyr Asp Glu Ile Gln Gln Glu Met Lys Arg Ala Lys Val Ser
   500 505 510
   Gln Ala Leu Phe Ala Lys Val Ala Ala Thr Lys Ser Gln Gly Trp Leu
   515 520 525
   Cys Glu Leu Leu Arg Trp Lys Glu Asp Pro Ser Pro Glu Asn Arg Thr
   530 535 540
   Leu Trp Glu Asn Leu Ser Met Ile Arg Arg Phe Leu Ser Leu Pro Gln
   545 550 555 560
   Pro Glu Arg Asp Ala Ile Tyr Glu Gln Glu Ser Asn Ala Val His His
   565 570 575
   His Gly Asp Arg Pro Pro His Ile Ile His Val Pro Ala Glu Gln Ile
   580 585 590
   Gln Gln Gln Gln Gln Gln Gln Gln Gln Gln Gln Gln Gln Gln Gln Ala
   595 600 605
   Pro Pro Pro Pro Gln Pro Gln Gln Gln Pro Gln Thr Gly Pro Arg Leu
   610 615 620
   Pro Pro Arg Gln Pro Thr Val Ala Ser Pro Ala Glu Ser Asp Glu Glu
   625 630 635 640
   Asn Arg Gln Lys Thr Arg Pro Arg Thr Lys Ile Ser Val Glu Ala Leu
   645 650 655
   Gly Ile Leu Gln Ser Phe Ile Gln Asp Val Gly Leu Tyr Pro Asp Glu
   660 665 670
   Glu Ala Ile Gln Thr Leu Ser Ala Gln Leu Asp Leu Pro Lys Tyr Thr
   675 680 685
   Ile Ile Lys Phe Phe Gln Asn Gln Arg Tyr Tyr Leu Lys His His Gly
   690 695 700
   Lys Leu Lys Asp Asn Ser Gly Leu Glu Val Asp Val Ala Glu Tyr Lys
   705 710 715 720
   Glu Glu Glu Leu Leu Lys Asp Leu Glu Glu Ser Val Gln Asp Lys Asn
   725 730 735
   Thr Asn Thr Leu Phe Ser Val Lys Leu Glu Glu Glu Leu Ser Val Glu
   740 745 750
   Gly Asn Thr Asp Ile Asn Thr Asp Leu Lys Asp
   755 760
<210> 3
   <211> 20
   <212> DNA
   <213> Artificial
<220>
   <223> primer
<400> 3
   ctaaggccaa ccgtgaaaag 20
<210> 4
   <211> 20
   <212> DNA
   <213> Artificial
<220>
   <223> primer
<400> 4
   accagaggca tacagggaca 20
<210> 5
   <211> 20
   <212> DNA
   <213> Artificial
<220>
   <223> primer
<400> 5
   accacacttc atgcatcagc 20
<210> 6
   <211> 18
   <212> DNA
   <213> Artificial
<220>
   <223> primer
<400> 6
   ccagtggcag cagaaggt 18
<210> 7
   <211> 18
   <212> DNA
   <213> Artificial
<220>
   <223> primer
<400> 7
   aggagtgccc cctttcac 18
<210> 8
   <211> 20
   <212> DNA
   <213> Artificial
<220>
   <223> primer
<400> 8
   tgctgctgag acatttgcat 20
<210> 9
   <211> 19
   <212> DNA
   <213> Artificial
<220>
   <223> primer
<400> 9
   caggaagcgg aaaaggagt 19
<210> 10
   <211> 23
   <212> DNA
   <213> Artificial
<220>
   <223> primer
<400> 10
   aaaattcaaa tagtgctggc aga 23
<210> 11
   <211> 25
   <212> DNA
   <213> Artificial
<220>
   <223> primer
<400> 11
   gctaccaaac tggatataat cagga 25
<210> 12
   <211> 24
   <212> DNA
   <213> Artificial
<220>
   <223> primer
<400> 12
   ccaggtagct atggtactcc agaa 24
<210> 13
   <211> 20
   <212> DNA
   <213> Artificial
<220>
   <223> primer
<400> 13
   cagggaatat ccaggaggaa 20
<210> 14
   <211> 22
   <212> DNA
   <213> Artificial
<220>
   <223> primer
<400> 14
   tgtgtgagcc agtgtgtatg aa 22
<210> 15
   <211> 20
   <212> DNA
   <213> Artificial
<220>
   <223> primer
<400> 15
   gaggtggcag aagagtggag 20
<210> 16
   <211> 20
   <212> DNA
   <213> Artificial
<220>
   <223> primer
<400> 16
   tgcatcacac tgcaaacaaa 20
<210> 17
   <211> 22
   <212> DNA
   <213> Artificial
<220>
   <223> primer
<400> 17
   gcagtagaaa ggtgggttct tc 22
<210> 18
   <211> 23
   <212> DNA
   <213> Artificial
<220>
   <223> primer
<400> 18
   tggtgacgaa agagaaataa atg 23
<210> 19
   <211> 24
   <212> DNA
   <213> Artificial
<220>
   <223> primer
<400> 19
   gaaaggtggg ttcttctgaa gata 24
<210> 20
   <211> 23
   <212> DNA
   <213> Artificial
<220>
   <223> primer
<400> 20
   gcaatgaatg cagaattacc ttt 23
<210> 21
   <211> 30
   <212> DNA
   <213> Artificial
<220>
   <223> primer
<400> 21
   gtatacagta tgcaaacata actcaccatt 30
<210> 22
   <211> 30
   <212> DNA
   <213> Artificial
<220>
   <223> primer
<400> 22
   aatggtgagt tatgtttgca tactgtatac 30
<210> 23
   <211> 30
   <212> DNA
   <213> Artificial
<220>
   <223> primer
<400> 23
   gtatacagta tcgtcgagca actcaccatt 30
<210> 24
   <211> 30
   <212> DNA
   <213> Artificial
<220>
   <223> primer
<400> 24
   aatggtgagt tgctcgacga tactgtatac 30
<210> 25
   <211> 30
   <212> DNA
   <213> Artificial
<220>
   <223> primer
<400> 25
   tcaaaaatat tgaagtgtta tcacatacac 30
<210> 26
   <211> 30
   <212> DNA
   <213> Artificial
<220>
   <223> primer
<400> 26
   gtgtatgtga taacacttca atatttttga 30
<210> 27
   <211> 20
   <212> DNA
   <213> Artificial
<220>
   <223> primer
<400> 27
   cgatgaactg aaacgagcag 20
<210> 28
   <211> 20
   <212> DNA
   <213> Artificial
<220>
   <223> primer
<400> 28
   cggaggattt ctgaaagcaa 20
<210> 29
   <211> 18
   <212> DNA
   <213> Artificial
<220>
   <223> primer
<400> 29
   acctacgcca cgctcatc 18
<210> 30
   <211> 19
   <212> DNA
   <213> Artificial
<220>
   <223> primer
<400> 30
   tcattgagtg tccgctgct 19
<210> 31
   <211> 20
   <212> DNA
   <213> Artificial
<220>
   <223> primer
<400> 31
   ggtttgttgc agccaaagat 20
<210> 32
   <211> 20
   <212> DNA
   <213> Artificial
<220>
   <223> primer
<400> 32
   tcttggccct cattctcact 20
<210> 33
   <211> 24
   <212> DNA
   <213> Artificial
<220>
   <223> primer
<400> 33
   cactgaagaa atctttcagg gaat 24
<210> 34
   <211> 22
   <212> DNA
   <213> Artificial
<220>
   <223> primer
<400> 34
   ccgtctttct tctccacact tt 22
<210> 35
   <211> 55
   <212> DNA
   <213> Homo sapiens
<400> 35
   ataaatggtg agttatgttt gcatactgta tactactcat agcaaattag agaag 55
<210> 36
   <211> 55
   <212> DNA
   <213> Pan troglodytes
<400> 36
   ataaatagtg agttatgttt gcatactgta tactactcgt agcaaattag agaag 55
<210> 37
   <211> 50
   <212> DNA
   <213> Mus musculus
<400> 37
   acaactgcag tgggttctgt ttgcgtagca gttaaagcaa atcagaccag 50
<210> 38
   <211> 57
   <212> DNA
   <213> Rattus norvegicus
<400> 38
   acaagtgcag agggttctgt ttgcgttctg tgtagcagtt agagcaaatc agaccag 57
<210> 39
   <211> 57
   <212> DNA
   <213> Canis familiaris
<400> 39
   acgcctatag tgagttctgt ttgcaccctg aataccactc acagcagatc agataag 57
<210> 40
   <211> 57
   <212> DNA
   <213> Bos taurus
<400> 40
   ccgagctgag tgaattacat ttgcacacca ggtaccactc acagcagatc agaccag 57
<210> 41
   <211> 60
   <212> DNA
   <213> Gallus gallus
<400> 41
   acatctgcag tgtgtattta ggttctcgtg ctgcctgcta ctcacaacaa atcaggcagg 60
<210> 42
   <211> 12
   <212> DNA
   <213> Artificial
<220>
   <223> FOXP3 consensus
<400> 42
   ttatgtttgc at 12
<210> 43
   <211> 20
   <212> DNA
   <213> Homo sapiens
<400> 43
   tgagttatgt ttgcatactg 20